# FASCICULE DE BREVET EUROPEEN

(11) **EP 1 537 087 B1**
(45) Date de publication et mention de la délivrance du brevet: **28.11.2012**
(21) Numéro de dépôt: 03769559.0
(22) Date de dépôt: 03.09.2003
(51) Int. Cl.: C07D 231/56, C07D 403/12, C07D 401/12, A61K 31/416, A61P 25/28, A61P 25/16, A61P 3/00, A61P 9/12, A61P 35/00, A61P 37/04

(54) **DERIVES DE 3-AMINOINDAZOLE COMME INHIBITEURS DE KINASE ET COMPOSITIONS PHARMACEUTIQUES LES RENFERMANT**
ALS KINASE-HEMMER WIRKSAME 3-AMINOINDAZOL-DERIVATE UND DIESE ENTHALTENDE PHARMAZEUTISCHE ZUSAMMENSETZUNGEN
NOVEL AMINOINDAZOLE DERIVATIVES AS MEDICINES AND PHARMACEUTICAL COMPOSITIONS CONTAINING SAME

(30) Priorité: 05.09.2002 FR 0210962; 22.10.2002 US 419965 P
(43) Date de publication de la demande: 08.06.2005
(73) Titulaire: Aventis Pharma S.A., 92160 Antony (FR)
(72) Inventeur: LESUISSE, Dominique, F-93100 Montreuil (FR); DUTRUC-ROSSET, Gilles, F-75012 Paris (FR); HALLEY, Franck, F-92310 Sevres (FR); BABIN, Didier, F-78180 Montigny (FR); ROONEY, Thomas, F-91400 Orsay (FR)
(74) Mandataire: Le Coupanec, Pascale A.M.P.
(86) Numéro de dépôt international: PCT/FR2003/002633
(87) Numéro de publication internationale: WO 2004/022544

(56) Documents cités:
- WO-A-02/22601
- WO-A-02/22603
- WO-A-02/50065
- WO-A-02/50066
- WO-A-03/078403

## Description

La présente invention concerne l'utilisation de dérivés de formule (I): 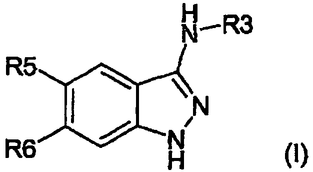 ou leurs sels pharmaceutiquement acceptables comme inhibiteur de kinase.

Les demandes de brevet WO 02/22603, WO 02/50065, WO 02/50066 et WO 02/22601 décrivent des dérivés de pyrazole ainsi que leur utilisation en tant qu'agents inhibiteurs de protéine kinase telles que GSK-3 et Aurora-2.

En outre, la demande de brevet WO 03/078403 (document tel que défini à l'article 54(3) CBE) décrit des dérivés d'aminoindazole ainsi que leur utilisation pour traiter des maladies dans lesquelles la phosphorylation de la protéine tau est observée.

L'invention a pour objet l'utilisation des dérivés d'aminoindazoles de formule (I) et leurs sels pharmaceutiquement acceptables pour la préparation de compositions pharmaceutiques destinées à prévenir et traiter les maladies pouvant résulter d'une activité anormale de kinases comme par exemple celles impliquées dans les maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, les maladies du métabolisme, le diabète de type II, l'hypertension essentielle, les maladies cardiovasculaires athérosclérotiques, le syndrome des ovaires polycystiques, le syndrome X, l'immunodéficience et le cancer, les compositions pharmaceutiques contenant les nouveaux dérivés d'aminoindazoles et leurs sels pharmaceutiquement acceptables et les dérivés nouveaux d'aminoindazoles et leurs sels pharmaceutiquement acceptables.

La présente invention concerne des dérivés de formule (I) dans laquelle :
R3 est un radical (1-6C)alkyle, aryle, aryle(1-6C)alkyle, hétéroaryle, hétéroaryle(1-6C)alkyle, aryle ou hétéroaryle fusionné à un cycloalkyle (1-10C), hétérocycle, hétérocycloalkyle, cycloalkyle, adamantyle, polycycloalkyles, alkényle, alkynyle, CONR1R2, CSNR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R¹, SO₂R1, NHSO₂R1, SO₂NR1R₂, C(S)NR1R₂, NHC(S)R1, -O-SO₂R1, -SO₂-O-R1, aryle, hétéroaryle, formyle, oxo, trifluorométhyle, trifluorométhylsulfanyle, trifluorométhoxy, (1-6C)alkyle ;
R5 est un aryle éventuellement substitué par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, bifluorométhyle, trifluorométhoxy, (1-6C)alkyle ;
R6 est un radical halogène, méthyle, cyclopropyle, CN, OH, méthoxy, trifluorométhyle, éthylényle, acétylényle, trifluomméthoxy, NO2, NH2, NMe2 ;
R1, R2, R10 et R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy ;
leurs racémiques, énantiomères, diastéréoisomères et leurs mélanges, leurs tautomères et leurs sels pharmaceutiquement acceptables.

De manière préférée, la présente invention concerne des dérivés de formule (I) dans laquelle :
R3 est un radical (1-6C)alkyle, aryle, aryle(1-6C)alkyle, hétéroaryle, hétéroaryle(1-6C)alkyle, aryle ou hétéroaryle fusionné à un cycloalkyle (1-10C), hétérocycle, hétérocycloalkyle, cycloalkyle, adamantyle, polycycloalkyles, alkényle, alkynyle, CONRIR2, CSNR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, oxo, trifluorométhyle, trifluoromethylsulfanyle, trifluorométhoxy, (1-6C)alkyle ;
R5 est un phényl éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifluorométhoxy, (1-6C)alkyle;
R6 est un radical halogène, méthyle, cyclopropyle, CN, OH, méthoxy, trifluorométhyle, éthylényle, acétylényle, trifluorométhoxy, NO2, NH2, NMe2 ;
R1, R2, R10 et R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NN₂, OH, COOH, COOalkyle, CONH₂, formyle, oxo, trifluorométhyle, trifluorométhoxy ;
leurs racémiques, énantiomères, diastéréoisomères et leurs mélanges, leurs tautomères et leurs sels pharmaceutiquement acceptables.

De manière préférée, la présente invention concerne des dérivés de formule (I) dans laquelle:
R3 est un radical (1-6C)alkyle, aryle, aryle(1-bC)alkyle, hétéroaryle, hétéroaryle(1-6C)alkyle, aryle ou hétéroaryle fusionné à un cycloalkyle (1-10C), hétérocycle, hétérocycloalkyle, cycloalkyle, adamantyle, polycycloalkyles, alkényle, alkynyle, CONR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, trifluorométhyle, trifluorométhylsulfanyle, trifluorométhoxy, (1-6C)alkyle ;
R5 est un phényle;
R6 est un chlore;
R1, R2 sont indépendamment l'un de l'autre un hydrogène, (1-6C)alkyle, aryle, alkényle, alkynyl, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ; leurs isomères, leurs mélanges, leurs racémiques, énantiomères, diastéréoisomères, tautomères ainsi que leurs sels pharmaceutiquement acceptables.

Dans les définitions précédentes et celles qui suivent, les radicaux alkyles (1-6C) contiennent 1 à 6 atomes de carbone en chaîne droite ou ramifiée; les radicaux alkényles contiennent 2 à 6 atomes de carbone et une à 3 doubles liaisons conjuguées ou non en chaîne droite ou ramifiée; les radicaux alkynyles contiennent 2 à 6 atomes de carbone et 1 à 3 triples liaisons conjuguées ou non en chaîne droite ou ramifiée ; les radicaux aryles sont choisis parmi phényle, naphtyle ou indényle ; les radicaux hétéroaryles contiennent 3 à 10 chaînons, contenant éventuellement un ou plusieurs hétéroatomes choisis parmi oxygène, soufre et azote en particulier, thiazolyle, thiényle, pyrrolyle, pyridinyle, furyle, imidazolyle, oxazolyle, pyrazinyle, tetrazolyle, oxadiazolyl, thiadiazolyle, isoxadiazolyl, isothiadiazolyl, isothiazolyle, isoxazolyle, triazolyle, pyrazolyle indolyle ; le radical halogène est soit, chlore, iode, fluor, brome ; les radicaux polycycloalkyles sont choisis parmi adamantyle, quinuclidinyle, bornanyle, norbornanyle, bornenyle, norbornenyle; les radicaux hétéroaryles fusionnés à un cycloalkyle (1-10C) sont choisi parmi indanyle, isochromanyle, chromanyle, 1,2,3,4-tétrahydroisoquinolyle, 1,2,3,4-tétrahydroquinolyle ; les radicaux hétérocycles contiennent 1 à 2 hétéroatomes choisis parmi oxygène, soufre, azote et représentent en particulier piperidinyle, morpholinyle, pyrrolidinyle, imidazolidinyle, pyrrazolidinyle, isothiazolidinyle, thiazolidinyle, isoxazolidinyle, oxazolidinyle, piperazinyle, azétidinyle, 2-piperidone, 3-piperidone, 4-piperidone, 2-pyrrolidone, 3-pyrrolidone.

Les composés de formule (I) présentent un ou plusieurs carbones asymétriques et peuvent donc se présenter sous forme d'isomères, de racémique, d'énantiomères et de diastéréoisomères; ceux-ci font également partie de l'invention ainsi que leurs mélanges.

Parmi les composés de formule (I) utiles selon l'invention on peut citer les composés suivants:
N-(bicyclo[2,2,1]hept-5-en-2ylmethyl)-6-chloro-5-phenyl-1H-indazol-3-amine
6-chloro-N-(3,3-dimethylbutyl)-5-phenyl-1H-indazol-3-amine
6-chloro-N-(3-phenylpropyl)-5-phenyl-1H-indazol-3-amine
6-chloro-N-(cyclopropylmethyl)-5-phenyl-1H-indazol-3-amine
6-chloro-N-(cyclopentylmethyl)-5-phenyl-1H-indazol-3-amine
6-chloro-N-[3-(methylthio)propyl]-5-phenyl-1H-indazol-3-amine
6-chloro-N-(phenylethyl)-5-phenyl-1H-indazol-3-amine
6-chloro-N-(cyclohexylmethyl)-5-phenyl-1H-indazol-3-amine
6-chloro-N-propyl-5-phenyl-1H-indazol-3-amine
6-chloro-N-(2,2,3,3,4,4,4-heptafluorobutyl)-5-phenyl-1H-indazol-3-amine, hydrate
6-chloro-N-(4,4,4-trifluorobutyl)-5-phenyl-1H-indazol-3-amine
6-chloro-N-[(4-methoxyphenyl)methyl]-5-phenyl-1H-indazol-3-amine
6-chloro-N-(phenylmethyl)-5-phenyl-1H-indazol-3-amine
6-chloro-N-[(4-cyanophenyl)methyl]-5-phenyl-1H-indazol-3-amine
N-[(4-chlorophenyl)methyl]-6-chloro-5-phenyl-1H-indazol-3-amine
6-chloro-N-[(3-methoxyphenyl)methyl]-5-phenyl-1H-indazol-3-amine
6-chloro-N-[[4-(trifluoromethoxy)phenyl]methyl]-5-phenyl-1H-indazol-3-amine
N-[4-[[[6-chloro-5-phenyl-1H-indazol-3-yl]amino]methyl]phenyl]-acetamide
6-chloro-N-[(3,5-dichlorophenyl)methyl]-5-phenyl-1H-indazol-3-amine
6-chloro-5-phenyl-N-[[[4-(trifluoromethyl)phenyl]methyl]-1H-indazol-3-amine
6-chloro-N-[(4-fluorophenyl)methyl]-5-phenyl-1H-indazol-3-amine
6-chloro-N-[3-(4-methylphenoxy)phenylmethyl]-5-phenyl-1H-indazol-3-amine
N-(2,2,3,3,4,4,4-heptafluorobutyl]-6-chloro-5-phenyl-1H-indazol-3-amine
6-chloro-5-phenyl-N-[[3,5-bis(trifluoromethyl)phenyl]methyl]-1H-indazol-3-amine
6-chloro-5-phenyl-N-[[3-(trifluoromethyl)phenyl]methyl]-1H-indazol-3-amine
6-chloro-N-[(6-methoxy-2-naphthalenyl)methyl]-5-phenyl-1H-indazol-3-amine
6-chloro-N-[(pentafluorophenyl)methyl]-5-phenyl-1H-indazol-3-amine
6-chloro-N-[[4-(methylthio)phenyl]methyl]-5-phenyl-1H-indazol-3-amine
N-[(4-chloro-3-auorophenyl)methyl]-6-chloro-5-phenyl-1H-indazol-3-amine
6-chloro-5-phenyl-N-(3,3,3-trifluoropropyl)-1H-indazol-3-amine
6-chloro-5-phenyl-N-(3-thienylmethyl)-1H-indazol-3-amine
N-(bicyclo[2,2,1]hept-5-en-2ylmethyl)-6-chloro-5-phenyl-1H-indazol-3-amine
N-([1,1'-biphenyl]-4-ylmethyl)-6-chloro-5-phenyl-1H-indazol-3-amine
6-chloro-N-[[4-(dimethylamino)phenyl]methyl]-5-phenyl-1H-indazol-3-amine
N-([2,2'-bithiophen]-5-ylmethyl)-6-chloro-5-phenyl-1H-indazol-3-amine
6-chloro-5-phenyl-N-[[1-(phenylmethyl)-1H-imidazol-2-yl]methyl]-1H-indazol-3-amine
6-chloro-N-[[1-methyl-1H-imidazol-2-yl]methyl]-5-phenyl-1H-indazol-3-amine
6-chloro-N-[(1-methyl-1H-indol-3-yl)methyl]-5-phenyl-1H-indazol-3-amine
6-chloro-N-[(5-methyl-2-furanyl)methyl]-5-phenyl-1H-indazol-3-amine
6-chloro-5-phenyl-N-(1H-pyrrol-2-ylmethyl)-1H-indazol-3-amine
6-chloro-5-phenyl-N-(1H-imidazol-2-yl)methyl]-1H-indazol-3-amine
6-chloro-5-phenyl-N-(1H-imidazol-4-yl)methyl]-1H-indazol-3-amine
6-chloro-5-phenyl-N-(1H-pyrazol-3-ylmethyl)-1H-indazol-3-amine
6-chloro-N-[[2-methyl-1H-imidazol-4-yl]methyl]-5-phenyl-1H-indazol-3-amine
6-chloro-N-[(3,5-dimethyl-1-phenyl-1H-pyrazol-4-yl)methyl]-5-phenyl-1H-indazol-3-amine
6-chloro-5-phenyl-N-[[2-phenyl-1H-imidazol-4-yl]methyl]-1H-indazol-3-amine
6-chloro-N-[[5-(4-chlorophenyl)-2-furanyl]methyl]-5-phenyl-1H-indazol-3-amine
6-chloro-5-phenyl-N-[(1-methyl-1H-pyrrol-2-yl)methyl]-1H-indazol-3-amine
4-[5-[[[6-chloro-5-phenyl-1H-indazol-3-yl]amino]methyl]-2-furanyl]-Benzenesulfonamide
6-chloro-5-phenyl-N-(3-thienylmethyl)-1H-indazol-3-amine
6-chloro-5-phenyl-N-[[2-phenyl-1H-imidazol-4-yl]methyl]-1H-indazol-3-amine
2-[[[6-chloro-5-phenyl-1H-indazol-3-yl]amino]methyl]-5-(methylthio)-1H-imidazole-4-carboxylate d'éthyle
6-chloro-5-phenyl-N-[[5-[4-(trifluoromethyl)phenyl]-2-furanyl]methyl]-1H-indazol-3-amine
6-chloro-5-phenyl-N-[2-(1-piperidinyl)ethyl]-1H-indazol-3-amine
6-chloro-N-[2-(4-morpholinyl)ethyl]-5-phenyl-1H-indazol-3-amine
N-(6-chloro-5-phenyl-1H-indazol-3-yl)-N'-(3,5-dichlorophenyl)- Urée
N-(6-chloro-5-phenyl-1H-indazol-3-yl)-N'-(2-propenyl)- Urée
N-(6-chloro-5-phenyl-1H-indazol-3-yl)-N'-(phenylmethyl)- Urée
N-(6-chloro-5-phenyl-1H-indazol-3-yl)-N'-4-(phenoxyphenyl)- Urée
N-(6-chloro-5-phenyl-1H-indazol-3-yl)-N'-[(4-methoxyphenyl)methyl]- Urée
N-(6-chloro-5-phenyl-1H-indazol-3-yl)-N'-[4-(trifluoromethyl)phenyl]- Urée
N-(6-chloro-5-phenyl-1H-indazol-3-yl)-N'-(4-methoxyphenyl)- Urée
N-(6-chloro-5-phenyl-1H-indazol-3-yl)-N'-cyclohexyl- Urée
N-(6-chloro-5-phenyl-1H-indazol-3-yl)-N'-propyl- Urée
N-(6-chloro-5-phenyl-1H-indazol-3-yl)-N'-(4-chlorophenyl)- Urée
N-(6-chloro-5-phenyl-1H-indazol-3-yl)-N'-(4-fluorophenyl)- Urée
N-[6-chloro-5-phenyl-1H-indazol-3-yl]-N'-tricyclo[3.3.1.13,7]dec-1-yl- Urée
N-(6-chloro-5-phenyl-1H-indazol-3-yl)-N'-(4-methylphenyl)- Urée
N-[6-chloro-5-phenyl-1H-indazol-3-yl]-4-methyl- Benzenesulfonamide
N-[6-chloro-5-phenyl-1H-indazol-3-yl]-Methanesulfonamide
N-[6-chloro-5-phenyl-1H-indazol-3-yl]-2-propanesulfonamide
N-[6-chloro-5-phenyl-1H-indazol-3-yl]-2,2,2-trifluoro-ethanesulfonamide
N-[6-chloro-5-phenyl-1H-indazol-3-yl]-2-thiophenesulfonamide
N-[6-chloro-5-phenyl-1H-indazol-3-yl]- Benzenesulfonamide
N-[6-chloro-5-phenyl-1H-indazol-3-yl]-4-(trifluoromethyl)-Benzenesulfonamide
N-[6-chloro-5-phenyl-1H-indazol-3-yl]-5-(3-isoxazolyl)-2-thiophenesulfonamide
N-[6-chloro-5-phenyl-1H-indazol-3-yl]-4-fluoro-Benzenesulfonamide
N-[6-chloro-5-phenyl-1H-indazol-3-yl]-4-methoxy-Benzenesulfonamide
N-[6-chloro-5-phenyl-1H-indazol-3-yl]-Benzenemethanesulfonamide
N-[6-chloro-5-phenyl-1H-indazol-3-yl]-1-methyl-1H-imidazole-4-sulfonamide
N-[6-chloro-5-phenyl-1H-indazol-3-yl]-4-(1,1-dimethylethyl)-Benzenesulfonamide
N-[4-[[(6-chloro-5-phenyl-1H-indazol-3-yl)amino]sulfonyl]phenyl]-Acetamide
N-[6-chloro-5-phenyl-1H-indazol-3-yl]-4-methyl-Benzenemethanesulfonamide
6-chloro-N-(pentafluorophenyl)-5-phenyl-1H-indazol-3-amine
6-chloro-N-(3,4-difluorophenyl)-5-phenyl-1H-indazol-3-amine
6-chloro-5-phenyl-N-(2,3,5,6-tetrafluorophenyl)-1H-indazol-3-amine
6-chloro-5-phenyl-N-(2,4,6-trifluorophenyl)-1H-indazol-3-amine
6-chloro-N-(4-fluorophenyl)-5-phenyl-1H-indazol-3-amine
6-chloro-N-[3-(trifluoromethyl)phenyl]-5-phenyl-1H-indazol-3-amine
6-chloro-N-[4-(trifluoromethyl)phenyl]-5-phenyl-1H-indazol-3-amine
6-chloro-N-[3-fluoro-5-(trifluoromethyl)phenyl]-5-phenyl-1H-indazol-3-amine
6-chloro-N-(4-nitrophenyl)-5-phenyl-1H-indazol-3-amine
6-chloro-N-(3-nitrophenyl)-5-phenyl-1H-indazol-3-amine
6-chloro-N-(3-methoxyphenyl)-5-phenyl-1H-indazol-3-amine
6-chloro-N-(4-methoxyphenyl)-5-phenyl-1H-indazol-3-amine
6-chloro-N,5-diphenyl-1H-indazol-3-amine
6-chloro-N-(1-pyridinyl)-5-phenyl-1H-indazol-3-amine
6-chloro-N-(2-pyridinyl)-5-phenyl-1H-indazol-3-amine
leurs isomères, leurs mélanges, leurs racémiques énantiomères, diastéréoisomères, tautomères ainsi que leurs sels pharmaceutiquement acceptables,
et plus particulièrement les composés suivants :
N-butyl-6-chloro-5-phenyl-1H-indazol-3-amine
3-(6-Chloro-5-phenyl-1H-indazol-3-ylamino)-thiophene-2-carbonitrile
(6-Chloro-5-phenyl-1H-indazol-3-yl)-pyridin-2-yl-amine
(6-Chloro-5-phenyl-1H-indazol-3-yl)-(5-nitro-pyridin-2-yl)-amine
(6-Chloro-5-phenyl-1H-indazol-3-yl)-(6-methoxy-pyridin-2-yl)-amine
N-(6-chloro-5-phenyl-1H-indazol-3-yl)-N'-phenyl- Urée
1-(6-Chloro-5-phenyl-1H-indazol-3-yl)-3-(4-ethoxy-phenyl)-urea
1-(6-Chloro-5-phenyl-1H-indazol-3-yl)-3-(3,4-dichloro-phenyl)-urea
3-[3-(6-Chloro-5-phenyl-1H-indazol-3-yl)-ureido]-propionic acid methyl ester
1-(6-Chloro-5-phenyl-1H-indazol-3-yl)-3-(4-dimthylamino-phenyl)-urea
1-(6-Chloro-5-phenyl-1H-indazol-3-yl)-3-isopropyl-urea
1-(6-Chloro-5-phenyl-1H-indazol-3-yl)-3-cyclohexyl-urea
1-(6-Chloro-5-phenyl-1H-indazol-3-yl)-3-(3-trifluoromethyl-phenyl)-urea
1-(6-Chloro-5-phenyl-1H-indazol-3-yl)-3-(2-thiophen-2-yl-ethyl)-urea
1-Benzo[1,3]dioxol-5-yl-3-(6-chloro-5-phenyl-1H-indazol-3-yl)-urea
1-(6-Chloro-5-phenyl-1H-indazol-3-yl)-3-(3,5-dimethyl-isoxazol-4-yl)-urea
1-Benzyl-3-(6-chloro-5-phenyl-1H-indazol-3-yl)-urea
1-(6-Chloro-5-phenyl-1H-indazol-3-yl)-3-phenethyl-thiourea
1-(6-Chloro-5-phenyl-1H-indazol-3-yl)-3-[3-(4-methyl-piperazin-1-yl)-propyl]-urea
1-(6-Chloro-5-phenyl-1H-indazol-3-yl)-3-(3-imidazol-1-yl-propyl)-urea
1-(6-Chloro-5-phenyl-1H-indazol-3-yl)-3-(hydroxy-ethyl)-urea
1-(6-Chloro-5-phenyl-1H-indazol-3-yl)-3-[3-(4-methyl-piperazin-1-yl)-propyl]-urea
(6-Chloro-5-phenyl-1H-indazol-3-yl)-carbamic acid methyl ester
(6-Chloro-5-phenyl-1H-indazol-3-yl)-urea
(6-Chloro-5-phenyi-1H-indazol-3-yl)-carbamic acid benzyl ester
(6-Chloro-5-phenyl-1H-indazol-3-yl)-carbamic acid allyl ester
(6-Chloro-5-phemyl-1H-mdazol-3-yl)-carbamic acid isobutyl ester
1-(3-Azetidin-1-yl-propyl)-3-(6-chloro-5-phenyl-1H-indazol-3-yl)-urea
1-(6-Chloro-5-phenyl-1H-indazol-3-yl)-3-(3-chloro-propyl)-urea
1-(6,7-Difluoro-5-phenyl-1H-indazol-3-yl)-3-(3-imidazol-1-yl-propyl)-urea
1-(3-Amino-propyl)-3-(6-chloro-5-phenyl-1H-indazol-3-yl)-urea
1-(6-Chloro-5-phenyl-1H-indazol-3-yl)-3-[4-(4-pyridin-3-yl-indazol-1-yl)-butyl]-urea
1-(6-Chloro-5-phenyl-1H-indazol-3-yl)-3-{2-pyrrolidin-3-yl-ethyl)-urea
N-(6-chloro-5-phényl-1H-indazol-3-yl-3-yl)-3-methoxy-benzénesulfonamide
leurs isomères, leurs mélanges, leurs racémiques énantiomères, diastéréoisomères, tautomères ainsi que leurs sels pharmaceutiquement acceptables,

L'invention concerne également les compositions pharmaceutiques contenant en tant que principe actif un dérivé de formule (I) dans laquelle
R3 est un radical (1-6C)alkyle, aryle, aryle(1-6C)alkyle, hétéroaryle, hétéroaryle(1-6C)alkyle, aryle ou hétéroaryle fusionné à un cycloalkyle (1-10C), hétérocycle, hétérocycloalkyle, cycloalkyle, adamantyle, polycyctoalkyles, alkényle, alkynyle, CONRIR2, CSNR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, oxo, trifluorométhyle, trifluorométhylsulfanyle, trifluorométhoxy, (1-6C)alkyle ;
R5 est un aryle éventuellement substitué par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NRSO₂R10, SO₂NR10R11, -O-SO₂R10, -SO₂-O-R10, aryle, hétéroaryle, formyle, trifluorométhyle, trifluorométhoxy, (1-6C)alkyle ;
R6 est un radical halogène, méthyle, cyclopropyle, CN, OH, méthoxy, trifluoraméthyle, éthylényle, acétylényle, trifluorométhoxy, N02, NH2, NMe2 ;
R1, R2, R10 et R11 sont indépendamment l'un de l'autre un hydrogène, (1-6C)alkyle, aryle, alkyle, alkynyle, hétéroaryle étant eux mêmes éventuellement substitués par 1 ou plusieurs substituants choisis parmi halogène, (1-6C)alkyle, (1-6C)alcoxy, CN, N0₂, NH₂, OH, COOH, COOalkyle, CONH₂, formyle, oxo, trifluorométhyle, trifluorométhoxy ;
leurs racémiques, énantiomères, diastéréoisomères et leurs mélanges, leurs tautomères et leurs sels pharmaceutiquement acceptables.

De manière préférée, la présente invention concerne les compositions pharmaceutiques contenant en tant que principe actif un dérivé de formule (I) dans laquelle :
R3 est un radical (1-6C)alkyle, aryle, aryle(1-6C)alkyle, hétéroaryle, hétéroaryle-(1-6C)alkyle, aryle ou hétéroaryle fusionné à un cycloalkyle (1-10C), hétérocycle, hétérocycloalkyle, cycloalkyle, adamantyle, polycycloalkyles, alkényle, alkynyl, CONR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, trifluorométhyle, trifluorométhylsulfanyle, trifluorométhoxy, (1-6C)alkyle ;
R5 est un phényle;
R6 est un chlore;
R1, R2 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formule, triflluororomethyle, trifluorométhoxy ; leurs isomères, leurs mélanges, leurs racémiques, énantiomères, diastéréoisomères, tautomères ainsi que leurs sels pharmaceutiquement acceptables.

La présente invention concerne également l'utilisation à titre de médicament des dérivés d'aminoindazoles de formule (I) dans laquelle :
R3 est un radical (1-6C)alkyle, aryle, aryle(1-6C)alkyle, hétéroaryle, hétéroaryle(1-6C)alkyle, aryle ou hétéroaryle fusionné à un cycloalkyle (1-10C), hétérocycle, hétérocycloalkyle, cycloalkyle, adamantyle, polycycloalkyles, alkényle, alkynyle, CONR1R2, CSNR1R2, COOR1, SO2R1 C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NRSO₂R1, SO₂NR1R2, C(S)NR1R2, NRC(S)R1, -O-SO₂R1, -SO₂-O-R1, aryle, hétéroaryle, formyle, oxo, trifluorométhyle, trifluorométhylsulfonyle, trifluorométhoxy, (1-6C)alkyle ;
R5 est un aryle éventuellement substitué par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NRSO₂R10, SO₂NR10R11, -O-SO₂R10, -SO₂-O-R10, aryle, hétéroaryle, formyle, trifluorométhyle, trifluorométhoxy, (1-6C)alkyle ;
R6 est un radical halogène, méthyle, cyclopropyle, CN, OH, méthoxy, trifluorométhyle, éthylényle, acétylényle, trifluorométhoxy, NO₂, NH2, NMe2 ;
R1, R2, R10 et R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH, OH, COOH, COOalkyle, CONH₂, formule, oxo, trifluorométhyle, trifluoraméthoxy ;
leurs racémiques, énantiomères, diastéréoisomères et leurs mélanges, leurs tautomères et leurs sels pharmaceutiquement acceptables.

De manière préférée, la présente invention concerne l'utilisation à titre de médicament des dérivés d'aminoindazoles de formule (I) dans laquelle :
R3 est un radical (1-6C)alkyle, aryle, aryle(1-6C)alkyle, hétéroaryle, hétéroaryle(1-6C)alkyle, aryle ou hétéroaryle fusionné à un cyaloalkyle (1-10C), hétérocycle, hététocycloalkyle, cycloalkyle, adamantyle, polycycloalkyles, alkényle, alkynyle, CONR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, trifluorométhyle, trifluorométhylsulfinyle, trifluorométhoxy, (1-6C)alkyle ;
R5 est un phényle;
R6 est un chlore ;
R1, R2 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ; leurs isomères, leurs mélanges, leurs racémiques, énantiomères, diastéréoisomères, tautomères ainsi que leurs sels phannaceutiquement acceptables.

Les dérives de formule (I) peuvent être obtenus à partir des dérivés 3-amino correspondants (V), pour lesquels l'azote en 1 est éventuellement protégé avec un groupement Pr. Pr est un radical triméthylsilyléthoxyméthyle, tosyle, mésyle, benzyle ou les groupements connus pour la protection des NH- d'hétérocycles aromatiques comme indiqués dans T.W. GREENE, Protective groups in organic Synthesis, J. Wiley-Interscience Publication (1999)

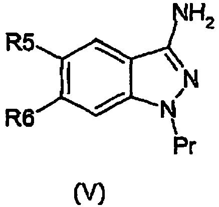

Les 3-amino 1H-indazoles de formule (II) peuvent être obtenus par réaction d'un 2-fluorobenzonitrile avec de l'hydrazine, hydrate ou chlorhydrate an reflux de 2 à 18 heures dans un alcool type méthanol ou n-butanol selon (R.F. KALTENBACH, Bioorg. Med. Chem. Lett., 9,(15), 2259-62, (999)):

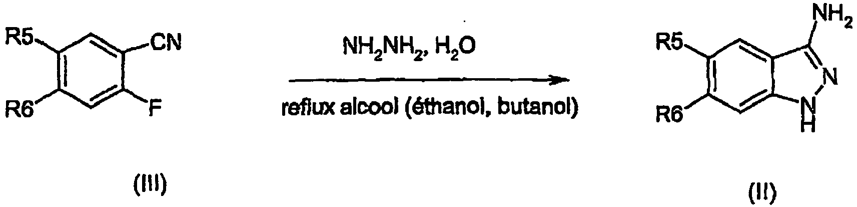

Pour les composés pour lesquels R5, R6 sont indépendamment l'un de l'autre choisis parmi les radicaux suivant hydrogène halogène, CN, NO₂, NH₂, OH, trifluoromkhyle, trifluorométhoxy, aryle, cyclopropyle, éthylényle, acétylényle, méthyle, méthoxy; NMe₂ ces radicaux étant éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifiuorométhoxy, (1-6C)alkyle; peuvent être obtenus par des réactions mettant en jeu la chimie du palladium : Suzuki, (A. SUZUKI, Pure Appl. Chem. 63, 419-22, (1991), Stille (J.. STILLE, Angew. Chem. Int. Ed. 25, 508-24, (1986), Heck, (R. F. HECK, Org. React., 27, 345-90, (1982), Sonogashira, (K. SONOGASHIRA, Synthesis 777, (1977), Buckwald (S.L. BUCKWALD, Acc. Chem.Re., 31, 805, (1998) à partir des dérivés halogénés correspondants.

Pour cela il est nécessaire de protéger les fonctions réactives. Ainsi, les fonctions OH, SH, COOH, NH2 doivent être protégées avant de faire le couplage. Les groupements protecteurs sont introduits selon toutes les méthodes connues de l'homme de l'art et notamment celles décrites par T.W. GREENE, Protective groups in Organic Synthesis, J. Wiley-Interscience Publication (1999). Il est préférable de protéger l'azote en position 1 par des groupements tels que le *tert*-butoxycarbonyle ou des dérivés siliciés. On choisira de préférence un groupement silylé *tert-*butyldiméthylsilyle, triisopropylsilyle qui peuvent être éliminés par les anions fluorure ou avec l'acide acétique et plus particulièrement un groupement triméthylsilyléthoxyméthyle clivable par le fluorure de tétrabutylammonium au reflux dans des solvants tels que le tétrahydrofurane, le dioxane (J. P. WHTTTEN, J. Org. Chem., 51, 1891, (1986) ; B. H. LIPSHUTZ, Tetrahedron Lett., 4095, (1986)) ou par l'acide chlorhydrique 2N dans le methanol ou l'ethanol au reflux.

Les dérivés protégés en 1 par triméthylsilyléthoxyméthyle sont obtenus en faisant réagir le composés de départ avec le chlorure de triméthylsilyléthoxyméthyle en présence d'hydrure de sodium dans un solvant tel que le diméhylformamide à température ambiante (J. P. WHITTEN, J. Org. Chem., 51, 1891, (1986) ; M. P. EDWARDS, Tetrahedron, 42, 3723, (1986))

De même, la fonction azote 1-NH de l'indazole sera protégée par des groupements tels tosyle, carbamate, benzyle ou dérivés silylés. Par exemple dans le cas où l'on voudrait pratiquer un couplage au palladium sur un dérivé halogéné en position 6, il faudra protéger l'azote en position 1 comme montré ci-dessous (X = Cl, Br, I) :

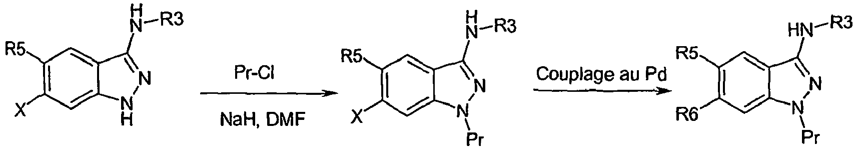

La déprotection s'effectue selon des méthodes connues par l'homme du métier et décrites par T.W. GREENE, Protective groups in Organic Synthesis, J. Wiley-Interscience Publication (1999). Par exemple, si le groupement protecteur en position 1 est un triméthylsilyléthoxyméthyle il pourra être déprotégé par réaction avec le fluorure de tétrabutylammonium comme montré ci-dessous:

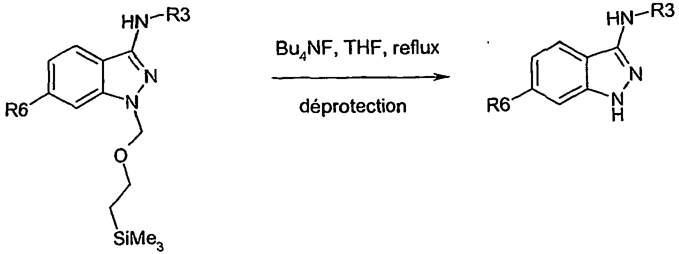

Lorsque l'un des groupements R5, R6 engagé pour le couplage utilisant la chimie du palladium contient lui-même une fonction réactive telle hydroxyle, amine, thiol, acide ou de manière générale renferme un hétéroatome, il est nécessaire de protéger ces dernières également avant d'effectuer le couplage au palladium. Ainsi par exemple une fonction phénol sera introduite sous la forme protégée (O-benzyle par exemple) à partir du dérivé chloré et l'azote en 1 étant protégé comme explicité auparavant :

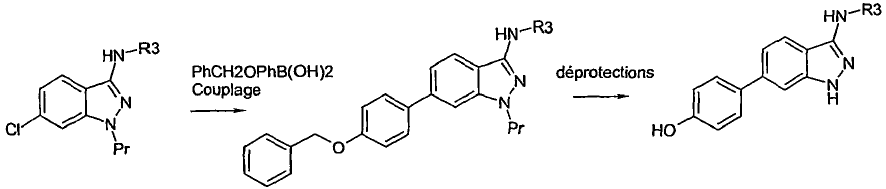

Le groupement benzyle sera ensuite éliminé par exemple par traitement à l'iodure de triméthylsilyle au reflux dans l'acétonitrile. La protection pourra également être réalisée par un groupement triméthylsilyléthoxyméthyle clivable par le fluorure de tétrabutylammonium au reflux dans des solvants tels que le tetrahydrofurane, le dioxane. (J. P. WHITTEN, J. Org. Chem., 51, 1891, (1986); B. H. LIPSHUTZ, Tetrahedron Lett., 4095, (1986)). ou par l'acide chlorhydrique 2N dans le methanol ou l'ethanol au reflux.

Lorsque R5 et R6 sont indépendamment l'un de l'autre un aryle et un halogène, la fonction aryle est introduite à partir d'un couplage au palladium sur une position bromée, l'azote en 1 et 3 étant protégé de manière appropriée. De préférence Pr représente un triméthylsilyléthoxyméthyle et Pr' représente un groupement n-butylcarboxy qui forme avec l'azote un n-butylamide. L'étape de déprotection de l'amide se fait en présence d'éthanolamine à reflux pendant une semaine dans la DMF. Ce clivage peut être aussi réalisé par le chlorure stanneux dans l'ethanol (R J Griffin, J.Chem.Soc. Perkin I 1992, 1811-1819) ou bien le methylate de sodium dans le methanol (Y. Furukawa, Chem.Pharm.Bull. 1968,16, 1076) ou tout autre alcoolate dans l'alcool correspondant.

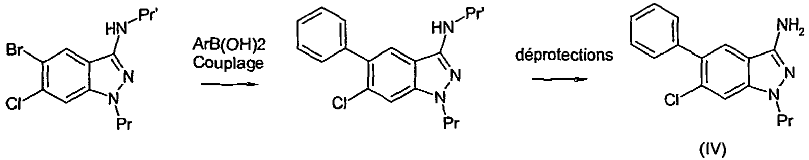

Les composés de formule II sont le point de départ pour l'obtention d'une grande variété de produits obtenus par réaction de la fonction amine primaire du 3-amino indazole dans toutes les réactions classiques de cette fonction telles que : alkylation, acylation, reactions avec les dérivés carbonylés suivit de réduction, sulfonation, transformation en urées ou carbamates, arylation (réactions de Castro ou de Buchwald) etc...

Les amino reduction de dérivés de dérivé formule générale (I) où R3 est H lorsque Pr est triméthylsilyléthoxyméthyle peuvent être réalisées à l'aide de dérivés du bore comme le triacétoxyborohydrure de sodium dans du dichlorométhane en présnece d'un aldéhyde de type R1CHO dans les conditions décrites dans Organic Reactions vol 59 1-714 ( E.Baxter, A.Reitz) ou par les autres reducteurs couramment utilisés pour réduire les imines pour former des produits où R3 est (1-6C)alkyle, aryle(1-6C)alkyle, hétéroaryle(1-6C)alkyle, hétérocycloalhyle, cycloalkyle, polycycloalkyles, ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, trifluorométhyle, trifluorométhylsulfanyle, trifluorométhoxy, (1-6C)alkyle.

Les condensations avec des dérivés formule générale (I) où R3 est H sur les isocyanates de type OCNR1 peuvent notamment être réalisées dans du tétrahydrofurane et selon les exemples décrits dans Comprehensive Organic functionnal Group Transformations vol 6 ( Katritzky, Meth-Cohn, Rees 1995) pour former des produits où R3 est CONR1R2, R1, R2, sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy.

Les sulfonations de dérivés de formule générale (I) où R3 est H peuvent être réalisées à partir d'un chorure de sulfonyle de type R1SO2Cl, en présence d'une base ( en particulier les amines tertiaires comme la triéthylamine ou aromatiques comme la pyridine) dans un solvant usuel comme par exemple le dichloromethane pour former les produits où R3 est SO2R1 et R1 est un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy.

Le composé IV avec Pr est triméthylsilyléthoxyméthyle est le 3-amino-5-phényl-6-chloro-1-(2-triméthylsilyléthoxy)méthyl]-indazole et est obtenu de la manière suivante :
3-amino-5-phenyl-6-chloro-1-(2-triméthylsilyléthoxy)méthyl]-indazole

A 2,4g de N-[[5-phenyl-6-chloro-1-(2-triméthylsilyléthoxy)méthyl]-indazo1-3-yl]]-butanamide décrit ci-après, dans 75 cm³ de diméthylformamide on ajoute 1,63 cm³ d'éthanolamine puis 2,24 g de carbonate de potassium et on chauffe au reflux pendant une semaine. Le milieu réactionnel est concentré à sec sous pression réduite et repris par 250 cm³ d'acétate d'éthyle et 100 cm³ d'eau. La phase organique est décantée et lavée successivement par 2 fois 100 cm³ d'eau et 75 cm³ de saumure. La phase organique est séchée sur sulfate de magnésium, filtrée puis concentrée à sec sous pression réduite (2kPa-50°C). L'huile brute obtenue est purifiée par chromatographie sous pression d'argon de 50 kPa, sur colonne de gel de silice (granulométrie 40-60 µm ; diamètre 4cm), en éluant par un mélange cyclohexane-acétate d'éthyle (80/20 en volumes) et en recueillant des fractions de 35 cm³. Les fractions contenant le produit attendu, sont réunies et évaporées sous pression réduite (2 kPa; 50°C). On obtient après séchage (90Pa-45°C), 0,43 g de 3-amino-5-phényl-6-chloro-1-(2-triméthylsilyléthoxy)méthyl]-indazole sous forme d'une huile jaune.

Spectre de R.M.N ¹H (300 MHz, (CD₃)₂SO d6, δ en ppm) : - 0,05 (s : 9H) ; 0,83 (t, J = 8 Hz : 2H) ; 3,52 (t, J = 8 Hz : 2H) ; 5,49 (s : 2H) ; 5,75 (s large : 2H) ; de 7,30 à 7,55 (mt : 5H) ; 7,77 (s : 1H) ; 7,81 (s : 1H).

Le N-[[5-phenyl-6-chloro-1-(2-triméthylsilyléthoxy)méthyl]-indazol-3-yl]]-butanamide est obtenu de la manière suivante :
A 2g de N-[[5-bromo-6-chloro-l-(2-triméthylsilyléthoxy)méthyl]-indazol-3-yl]]-butanamide décrit ci-après dans 180 cm³ de dioxanne, on ajoute 821 mg d'acide phénylboronique, 1,14 g de carbonate de sodium dans 30 cm³ d'eau distillée et enfin 347 mg de tétrakis( triphénylphosphine) palladium. On chauffe au reflux pendant 90 minutes puis on laisse revenir à 20 °C pour ajouter 100 cm³ d'acétate d'éthyle et 100 cm³ d'eau distillée. La phase organique est lavée avec 100 cm³ d'une solution aqueuse saturée en chlorure de sodium puis décantée et sèchée sur sulfate de magnésium. Après filtration sur verre fritté, le filtrat est concentré à sec sous pression réduite (2kPa-50°C). Le résidu est purifié par chromatographie sous pression d'argon de 50 kPa, sur colonne de gel de silice ( granulométrie 40-60 µm ; diamètre 4.5 cm), en éluant par un mélange cyclohexane-acétate d'éthyle ( 80/20 en volumes) et en recueillant des fractions de 35 cm³. Les fractions contenant le produit attendu sont réunies et évaporées sous pression réduite (2 kPa; 50°C). On obtient ainsi après séchage (90Pa-45°C), 2 g de N-[[5-phenyl-6-chloro-1-(2-triméthylsilyléthoxy)méthyl]-indazol-3-yl]]-butanamide sous forme d'une huile jaune.

Spectre de R.M.N ¹H (300 MHz, (CD₃)₂SO d6, δ en ppm) : - 0,05 (s : 9H) ; 0,85 (t, J = 8 Hz : 2H) ; 0,92 (t, J = 7,5 Hz : 3H) ; 1,63 (mt : 2H) ; 2,38 (t, J = 7,5 Hz : 2H) ; 3,56 (t, J = 8 Hz : 2H) ; 5,70 (s : 2H) ; de 7,30 à 7,55 (mt : 5H) ; 7,91 (s : 1H) ; 7,99 (s : 1H) ; 10,59 (s large : 1H).

Le N-[[5-bromo-6-chloro-1-(2-triméthylsilyléthoxy)méthyl]-indazol-3-yl]]-butanamide est obtenu de la manière suivante :
A 1g de N-[[6-chloro-1-(2-triméthylsilyléthoxy)méthyl]-indazol-3-yl]]-butanamide décrit ci-après dans 15 cm³ de chloroforme, on ajoute 0.22 cm³ de pyridine puis on additionne 0.14 cm³ de brome. On agite 24 heures à 20 °C puis on ajoute ensuite 50 cm³ de dichlorométhane et 50 cm³ d'une solution aqueuse saturée en sulfate de sodium. Après 10 minutes d'agitation l'insoluble est éliminé par filtration sur verre fritté et la phase organique est lavée avec 50 cm³ d'une solution aqueuse saturée en chlorure de sodium. La phase organique est décantée, sèchée sur sulfate de magnésium, filtrée et évaporée à sec sous pression réduite (2 kPa ; 45 °C). Le résidu est purifié par chromatographie sous pression d'argon de 50 kPa, sur colonne de gel de silice ( granulométrie 40-60 µm ; diamètre 3.5 cm), en éluant par un mélange acétate d'éthyle-cyclohexane (20/80 en volumes) et en recueillant des fractions de 35 cm³. Les fractions contenant le produit attendu, sont réunies et évaporées sous pression réduite (2 kPa ; 50°C). On obtient après séchage (90Pa-45°C), 0,94 g de N-[[5-bromo-6-chloro-1-(2-triméthylsilyléthoxy)méthyl]-indazol-3-yl]]-butanamide sous forme d'un solide blanc fondant à 130 °C.

Spectre de R.M.N ¹H (300 MHz, (CD₃)₂SO d6, δ en ppm) : - 0,08 (s : 9H) ; 0,82 (t, J = 8 Hz : 2H) ; 0,95 (t, J = 7,5 Hz : 3H) ; 1,66 (mt : 2H) ; 2,40 (t, J = 7,5 Hz : 2H) ; 3,52 (t, J = 8 Hz : 2H) ; 5,66 (s : 2H) ; 8,13 (s : 1H) ; 8,34 (s : 1H) ; 10,67 (s large : 1H).

Le N-[[6-chloro-1-(2-triméthylsilyléthoxy)méthyl]-indazol-3-yl]]-butanamide est obtenu de la manière suivante :
A 606 mg d'hydrure de sodium à 60%, dans 20 cm³ de diméthylformamide, on additionne 3g de N-(6-chloro-1H-indazol-3-yl)-butanamide en solution dans 40 cm³ de diméthylformamide. Après avoir refroidit vers 5°C, on ajoute 2.68cm³ de chlorure de 2-(triméthylsilyl)-éthoxyméthyle dans 10 cm³ de diméthylformamide. On laisse la température revenir vers 21°C et agite pendant 2 heures. Le milieu réactionnel est ensuite évaporé sous pression réduite (2kPa ; 45 °C). Le résidu est repris par 200 cm³ d'acétate d'éthyle et par 100 cm³ d'eau distillée. On relave avec 2 fois 100 cm³ d'eau distillée et avec 100 cm³ d'une solution aqueuse saturée en chlorure de sodium. La phase organique est séchée sur sulfate de magnésium, filtrée sur verre fritté puis évaporée sous pression réduite ( 2 kPa; 50°C). Le résidu est purifié par chromatographie sous pression d'argon de 50 kPa, sur colonne de gel de silice ( granulométrie 40-60 µm ; diamètre 4.5 cm), en éluant par un mélange cyclohexane-acétate d'éthyle (80/20 en volumes) et en recueillant des fractions de 100 cm³. Les fractions contenant le produit attendu sont réunies et évaporées sous pression réduite ( 2 kPa ; 50°C) . On obtient après séchage ( 90 Pa ; 50°C), 3 g de N-[[6-chloro-1-(2-triméthylsilyléthoxy)méthyl]-indazol-3-yl]]-butanamide sous forme d'une huile jaune.

Spectre de R.M.N. ¹H (300 MHz, (CD₃)₂SO d6, δ en ppm) : - 0,08 (s : 9H) ;.0,83 (t large, J = 8 Hz : 2H) ; 0,96 (t, J = 7,5 Hz : 3H) ; 1,67 (mt : 2H) ; 2,40 (t, J = 7,5 Hz : 2H) ; 3,53 (t, J = 8 Hz : 2H) ; 5,66 (s : 2H) ; 7,16 (dd, J = 9 et 2 Hz : 1H) ; 7,86 (d, J = 2 Hz : 1H) ; 7,88 (d, J = 9 Hz : 1H) ; 10,53 (mf : 1H).

### N-(6-chloro-1H-indazol-3-yl)-butanamide

A 750 mg de 3-amino-6-chloro-1H-indazole dans 10 cm³ de pyridine, on ajoute 0.47 cm³ de chlorure de butyryle, après avoir refroidit le milieu réactionnel vers 3°C. Puis on laisse ensuite revenir le milieu à 19 °C pendant 14 heures. Le milieu réactionnel est évaporé à sec sous pression réduite ( 2 kPa ; 40°C). Le résidu est repris par 50 cm³ d'acétate d'éthyle, par 50 cm³ de tétrahydrofurane et par 50 cm³ d'eau distillée. La phase organique est relavée avec 50 cm³ d'eau distillée et avec 50 cm³ d'une solution aqueuse saturée en chlorure de sodium puis séchée sur sulfate de magnésium, filtrée sur verre fritté et évaporée sous pression réduite. Le résidu obtenu est purifié par chromatographie sous une pression d'argon de 50 kPa, sur une colonne de gel de silice (granulométrie 40-60 µm; diamètre 2.5 cm), en éluant par cyclohexane-acétate d'éthyle (70/30 en volumes) et en recueillant des fractions de 25 cm³. Les fractions contenant le produit attendu sont réunies puis évaporées sous pression réduite ( 2 kPa ; 40 °C). On obtient après séchage ( 90 P ; 45°C), 200 mg de N-(6-chloro-1H-indazol-3-yl)-butanamide, sous forme d'un solide blanc fondant à 230°C.

Spectre de R.M.N. ¹H (300 MHz, (CD₃)₂SO d6, δ en ppm) : 0,98 (t, J = 7 Hz : 3H) ; 1,67 (mt : 2H) ; 2,40 (t, J = 7 Hz : 2H) ; 7,08 (dd, J = 9 et 2 Hz : 1H) ; 7,52 (d, J = 2 Hz : 1H) ; 7,84 (d, J = 9 Hz : 1H) ; 10,39 (mf : 1H) ; de 12,50 à 13,00 (mf étalé : 1H). Le 3-amino-6-chloro-5-phényl-1H-indazole-est obtenu à partir du 3-amino-5-phenyl-6-chloro-1-(2-triméthylsilyléthoxy)méthyl]-indazole.

A 108.3 mg de composé 3-amino-5-phenyl-6-chloro-1-(2-triméthylsilyléthoxy)méthyl]-indazole dans 4.7 ml de méthanol on ajoute 300 µl d'HCl 2N. La réaction est placée sous micro-ondes 150 secondes à 140°C.

On verse sur une solution de KH2PO4 saturée et on extrait à l'AcOEt. On sèche les phases organiques sur MgSO4 anhydre, on filtre et on concentre. Le brut obtenu est purifié sur silice et on obtient 63.5 mg de composé 3-amino-6-chloro-5-phényl-1H-indazole.

Les composés de formule (I) sont isolés et peuvent être purifiés par les méthodes connues habituelles, par exemple par cristallisation, chromatographie ou extraction.

Les composés de formule (I) peuvent être éventuellement transformés en sels d'addition avec un acide minéral ou organique par action d'un tel acide au sein d'un solvant organique tel qu'un alcool, une cétone, un éther ou un solvant chloré. Ces sels font également partie de l'invention.

Comme exemples de sels pharmaceutiquement acceptables, peuvent être cités les sels suivants : benzènesulfonate, bromhydrate, chlorhydrate, citrate, éthanesulfonate, fumarate, gluconate, iodate, maléate, iséthionate, méthanesulfonate, méthylène-bis-b-oxynaphtoate, nitrate, oxalate, pamoate, phosphate, salicylate, succinate, sulfate, tartrate, théophyllinacétate et p-toluènesulfonate.

Les composés de formule (I) sont des inhibiteurs de kinase et sont ainsi utiles pour la prévention et le traitement les maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, l'hypertension essentielle, les maladies cardiovasculaires athérosclérotiques, le syndrome des ovaires polycystiques, le syndrome X, l'immunodéficience et le cancer.

Leurs activités ont été déterminées en mesurant l'inhibition de la phosphorylation de la protéine tau dans les coupes de cortex de rat adulte.

Les coupes de cortex d'une épaisseur de 300µm sont préparées à partir de rats mâles OFA (Iffa-Credo) âgés de 8-10 semaines, sacrifiés par décapitation. Elles sont incubées dans 5 ml de milieu DMEM contenant du pyruvate et du glucose 4.5 g/l à 37°C pendant 40 min. Les coupes sont ensuite lavées 2 fois avec le milieu, distribuées dans des microtubes (50µl dans 500µl de milieu avec ou sans composés à tester), et incubées à 37°C sous agitation. Deux heures plus tard, l'expérience est arrêtée par centrifugation. Les coupes sont lysées, sonifiées et centrifugées à 18300g, 15 min à 4°C. La concentration en protéines du surnageant est déterminée par un dosage commercial (BCA Protein Assay , Pierce) basé sur la méthode de Lowry.

Les échantillons, dénaturés au préalable 10 min à 70°C, sont séparés sur gel vertical 4-12%Bis-Tris en présence de tampon MOPS-SDS et électrotansferrés sur membrane de nitrocellulose. L'immunomarquage est réalisé par l'anticorps monoclonal AD2 qui reconnaît spécifiquement les épitopes phosphorylés Ser396/404 de la protéine tau. Les protéines immunoréactives sont visualisées par addition d'un deuxième anticorps dirigé contre les IgG de souris et couplé à la peroxydase et d'un substrat chimioluminescent. Les autoradiogrammes obtenus sont enfin quantifiés à l'aide du logiciel 'GeneTools' de Syngene ( GeneGnome, Ozyme) pour déterminer une CI50.

Les composés de formule (I) présentent une activité très intéressante et en particulier certains composés ont une CI50 inférieure à 100 µM.

Les comditions d'analyse des produits en LC/MS ont été réalisées sur un appareil Waters Alliance 2695 pour la partie LC et Waters-Micromass Platform II pour la partie masse.

Les exemples suivants illustrent l'invention de manière non limitative.

### Exemple A1 : N-butyl-6-chloro-5-phenyl-1H-indazol-3-amine

*Etape 1 :* 24 mg de n-butyraldéhyde et 113 mg de triacétoxyborohydrure de sodium sont additionnés à une solution de 100 mg de 3-amino-5-phenyl-6-chloro-1-(2-triméthylsilyléthoxy)méthyl]-indazole dans 5cm³ de chlorure de méthylène. Après 3 heures à température ambiante, le milieu réactionnel est hydrolysé puis extrait au chlorure de méthylène. La phase organique est séchée sur sulfate de magnésium, filtrée et évaporée. La purification du brut par chromatographie sur silice (éluant : acétate d'éthyle/hexane (80/20, v/v)) permet d'obtenir 21mg de butyl-[6-chloro-5-phényl-1-(2-triméthylsilanylaaa-éthoxyméthyl)-1H-indazol-3-yl]-amine (solide jaune).

Spectre de masse : 432 [M+H]⁺ ; temps de rétention : 5.26 minutes.

RMN¹H [DMSO-d6] : 7.83 (1H, s) ; 7.73 (1H, s) ; 7.35-7.50 (5H, m) ; 6.25 (1H, t, J=6Hz) ; 5.49 (2H, s) ; 3.52 (2H, t, J=8Hz) ; 3.24 (2H, m) ; 1.60 (2H, m) ; 1.39 (2H, m) ; 0.91 (3H, t, J=7Hz) ; 0.81 (2H, t, J=8Hz) ; -0.07 (9H, s).

*Etape 2 :* 0.7 ml de HCl 2N est additionné à une solution de 21mg de butyl-[6-chloro-5-phényl-1-(2-triméthylsilanyl-éthoxyméthyl)-1H-indazol-3-yl]-amine dans 0.3cm³ de méthanol. Le milieu réactionnel est agité à température ambiante pendant 48 heures, 1 heure à reflux puis évaporé. Le solide obtenu est séché sous vide pour donner 16 mg de N-butyl-6-chloro-5-phenyl-1H-indazol-3-amine (solide jaune).

Spectre de masse : 300[M+H]⁺ ; temps de rétention : 4.25 minutes.

RMN¹H [DMSO-d6] : 7.52 (1H, s) ; 7.95(1H, s) ; 7.35-7.50 (5H, m) ; 3.30 (2H, t, J=7Hz) ; 1.61 (2H, m) ; 1.40 (2H, m) ; 0.92 (3H, t, J=7Hz).

Exemple A2 : 3-(6-Chloro-5-phenyl-1H-indazol-3-ylamino)-thiophene-2-carbonitrile A 52 mg de composé 3-amino-5-phenyl-6-chloro-l-[(2-triméthylsilyléthoxy)méthyl]-indazole dans 0.5 ml de NMP (1-methyl-2-pyrrolidone), sont ajoutés 38 mg de 2-dicyclohexylphosphino-2'-(N,N-dimethylamino) biphenyl , 20 mg de Pd2dba3 (tris(dibenzylideneacetone)dipalladium(0)), 52 mg de 2-cyano 3-bromo thiophene, 23 mg de tertbutylate de sodium.

La réaction est placée sous micro ondes pendant 3 mn à 140°C. Après traitements habituels, le brut est traité par HCl 2N dans le méthanol pour donner après purification 8.4 mg de 3-(6-Chloro-5-phenyl-1H-indazol-3-ylamino)-thiophene-2-carbonitrile.

Spectre de masse : 351[M+H]⁺; temps de rétention : 4.19 minutes.

RMN¹H [DMSO-d6] :7,40 (1H,m); 7,48 (2H,m); 7,53 (3H,m); 7,81 (1H,s); 8,09 (1H,s); 8,27 (1H,d,J=5,5 Hz,);8,91 (2H,s).

### Exemples A3 à A5

Les exemples ci dessous ont été obtenus de manière équivalente à A2

| N° | Nom | Produit de départ | Temps de rétention/ [M+H]⁺ | RMN DMSO-d6 sauf indication contraire |
|---|---|---|---|---|
| A3 | (6-Chloro-5-phenyl-1H-indazol-3-yl)-pyridin-2-yl-amine | 2-bromo pyridine | 2.89/ 321 | 7,07 ppm (1, 1H) ; de 7,36 ppm à 7,50 ppm (m, 5H) ; 7,55 ppm (1, 1H) ; 7,67 ppm (s, 1H) ; 7,87 ppm (s, 1H) ; 7,95 ppm (1, 1H) ; 8,21 ppm (1, 1H) dans MeOD |
| A4 | (6-Chloro-5-phenyl-1H-indazol-3-yl)-(5-nitro-pyridin-2-yl)-amine | 2-bromo 5-nitro pyridine | 4.58/ 366 | De 7,42 à 7,52 ppm (m,5H); 7,63 ppm (s,1H) ; 7,65 ppm (s,1H) ; 7,94 ppm (d, J=9Hz,1H) ; 8,43 ppm (dd, J=2,5-9 Hz, 1H) ; 9,13 ppm (d,J=2,5Hz, 1H) ; 7,77 ppm (s1,1H) ; 9,55 ppm (s1,1H) |
| A5 | (6-Chloro-5-phenyl-1H-indazol-3-yl)-(6-methoxy-pyridin-2-yl)-amine | 2-bromo 6-methoxy pyridine | | 3.87 ppm (s, 3H) ; 6.31 ppm (d, J = 7.5 Hz, 1H) ; 7.36 ppm (dl, J = 7.5 Hz, 1H) ; de 7.35 à 7.50 ppm (m, 5H) ; 7.36 ppm (t, J = 7.5 Hz,1H) ; 7.54 ppm (s, 1H) ; 7.69 ppm (sl, 1H) dans CDCl3 |

### Exemple B1 : N-(6-chloro-5-phenyl-1H-indazol-3-yl)-N'-phenyl- Urée

*Etape 1 :* 39 µl d'isocyanate de phényle sont additionnés à une solution de 102.2 mg de 3-amino-5-phenyl-6-chloro-1-(2-triméthylsilyléthoxy)méthyl]-indazole dans 2.5cm³ de tétrahydrofurane. Le milieu réactionnel est agité pendant 24 heures à température ambiante puis évaporé. La purification du brut par chromatographie sur silice (éluant : chlorure de méthylène/acétone (98/2, v/v)) permet d'obtenir 122.5mg de 1-[6-chloro-5-phényl-1-(2-triméthylsilanyl-éthoxyméthyl)-1H-indazol-3-yl]-3-phényl-urée (solide incolore).

Spectre de masse : 493[M+H]⁺ ; temps de rétention : 6.02 minutes.

RMN¹H [DMSO-d6] : 9.89 (1H, s large) ; 9.86(1H, s large) ; 8.20 (1H, s) ; 8.07 (1H, s) ; 7.35-7.50 (5H, m) ; 5.81 (2H, s) ; 3.66 (2H, t, J=8Hz) ; 0.92 (2H, t, J=8Hz) ; -0.12 (9H, s).

*Etape 2 :* 1 ml de HCl 2N est additionnéà une solution de 106mg de 1-[6-chloro-5-phényl-1-(2-triméthylsilanyl-éthoxyméthyl)-1H-indazol-3-yl]-3-phényl-urée dans 12cm³ de méthanol. Le milieu réactionnel est agité à température ambiante pendant 48 heures, 5 heures à reflux puis évaporé. Le solide obtenu est séché sous vide pour donner 82 mg de N-(6-chloro-5-phenyl-1H-indazol-3-yl)-N'-phenyl- Urée (solide incolore).

Spectre de masse : 363[M+H]⁺; temps de rétention : 5.15 minutes.

RMN¹H [DMSO-d6] : 12.64 (1H, s large) ; 9.70(1H, s large) ; 9.59 (1H, s large) ; 8.07 (1H, s) ; 7.64 (1H, s) ; 7.50 (7H, m) ; 7.30 (2H, m) ; 7.0 (1H, m).

### Exemple B2 : 1-(6-Chloro-5-phenyl-1H-indazol-3-yl)-3-(4-ethoxy-phenyl)-urea

A 80 mg de 3-amino-5-phenyl-6-chloro-1-(2-triméthylsilyléthoxy)méthyl]-indazole dans 1 ml de THF sont ajoutés 36.4 mg de 4-ethoxyphenyle isocyanate. On chauffe à 50°C 1 h puis on hydrolyse dans une solution de KH2PO4 saturée et extrait au chlorure de méthylène. Après séchage et évaporation, le brut est purifié par chromatographie sur silice avec un mélange AcOEt/hexane. Le produit obtenu est déprotégé dans 2 ml d'un mélange 1/1 MeOH/HCl 2N 3h au reflux. On obtient 62.5 mg de 1-(6-Chloro-5-phenyl-1H-indazol-3-yl)-3-(4-ethoxy-phenyl)-urea.

Spectre de masse : 407 [M+H]⁺; temps de rétention : 4.36

RMN¹H [DMSO-d6] : 1,3(3H,t,J=7 Hz); 3,98(2H,q,J=7Hz) ; 6,87 et 7,36 (AA'-BB', 4H) ; 7,36-7,50(5H,m) ; 7,63(1H,s) ; 8,08(1H,s) ;9,53(2H,s);12,53(1H,s)

### Exemples B3 à B12 :

Les produit B3 à B12 sont obtenus de manière équivalente au produit B2

| N° | Nom | Produit de départ | Temps de rétention/ [M+H]⁺ | RMN DMSO-d6 |
|---|---|---|---|---|
| B3 | 1-(6-Chloro-5-phenyl-1H-indazol-3-yl)-3-(3,4-dichloro-phenyl)-urea | 3,4 dichlorophenyle isocyanate | 4.75/ 407[M-H] | de 7,38 à 7,48 ppm (m, 6H) ; 7,53 ppm (d,J = 8,5 Hz, 1H) ; 7,66 ppm (s, 1H) ; 7,90 ppm (d,J = 2,5 Hz, 1H) ; 8,01 ppm (s, 1H) ; 9,70 ppm (s, 1H) ; 9,84 ppm (s, 1H) ; 12,72 ppm (s1, 1H) |
| B4 | 3-[3-(6-Chloro-5-phenyl-1H-indazol-3-yl)-ureido]-propionic acid methyl ester | Phenethyle isocyanate | 3.71/ 373 | 2,56 ppm (t,J = 6,5 Hz, 2H) ; 3,44 ppm (m, 2H); 3;61 (s,3H) ; de 7,36 à 7,50 ppm (m, 5H) ; 7,58 ppm (s, 1H) ; 7,81 (1, 1H) ; 8,08 ppm (s, 1H) ; 9,48 ppm (s, 1H) ; 12,52 ppm (s1, 1H) |
| B5 | 1-(6-Chloro-5-phenyl-1H-indazol-3-yl)-3-(4-dimethylamino-phenyl)-urea | 4-dimethyl-amino-phenyliso-cyanate | 3.26/ 406 | 3,10 ppm (s, 6H) ; de 7,38 à 7,50 ppm (m, 5H) ; 7,64 (1, 4H) ; 7,66 ppm (s, 1H) ; 8,03 ppm (s, 1H) ; 9,70 ppm (s, 1H) ; 9,95 ppm (s, 1H) ; 12, 72 ppm (s1, 1H) |
| B6 | 1-(6-Chloro-5-phenyl-1H-indazol-3-yl)-3-isopropyl-urea | Isopropyl isocyanate | 3.95 329 | 1,16 ppm (d,J = 6,5 Hz, 6H) ; 3;85 ppm (m, 1H) ; de 7,38 à 7,50 ppm (m, 5H) ; 7,58 ppm (1, 1H) ; 7,60 ppm (s, 1H) ; 8,10 ppm (s, 1H) ; 9,36 ppm (s, 1H) ; 12,48 ppm (s1, 1H) |
| B7 | 1-(6-Chloro-5-phenyl-1H-indazol-3-yl)-3-cyclohexyl-urea | Cyclohexyl-isocyanate | 4.37/ 369 | de 1,3 à 1,9 ppm (m, 10H) ; 3;58 ppm (m, 1H) ; de 7,38 à 7,49 ppm (m, 5H) ; 7,57 ppm (s, 1H) ; 7,68 ppm (dl,J= 5,5 Hz, 1H) ; 8,10 ppm (s, 1H) ; 9,38 ppm (s, 1H) ; 12,48 ppm (s1, 1H) |
| B8 | 1-(6-Chloro-5-phenyl-1H-indazol-3-yl)-3-(3-trifluoromethyl-phenyl)-urea | 3-trifluoro-methyl-phenyl isocyanate | 4.61/ 431 | 7,34 ppm (dl,J = 8 Hz, 1H) ; de 7,38 ppm à 7,49 ppm (m, 5H) ; 7,53 ppm (t,J = 8 Hz, 1H) ; 7,66 ppm (s, 1H) ; 7,69 ppm (dl,J = 8 Hz, 1H) ; 7,98 ppm (s1, 1H) ; 8,03 ppm (s, 1H) ; 9,71 ppm (s, 1H) ; 9,96 ppm (s, 1H) ; 12,76 ppm (s1, 1H) |
| B9 | 1-(6-Chloro-5-phenyl-1H-indazol-3-yl)-3-(2-thiophen-2-yl-ethyl)-urea | 2-thiophen-2-yl-ethyl isocyanate | 4.2/ 397 | 3,02 ppm (t,J = 7 Hz, 2H) ; 3;46 ppm (m, 2H) ; 6,92 ppm (dd,J = 1,5 - 3,5 Hz, 1H) ; 6,95 ppm dd,J = 3,5 - 5 Hz, 1H) ; 7,32 ppm (dd,J = 1,5 - 5 Hz, 1H) ; de 7,37 à 7,49 ppm (m, 5H) ; 7,58 ppm (s, 1H) ; 7,80 ppm (tl,J = 6 Hz, 1H) ; 8,08 ppm (s, 1H) ; 9,50 ppm (s, 1H) ; 12,48 ppm (s1, 1 H) |
| B10 | 1-Benzo[1,3]diox ol-5-yl-3-(6-chloro-5-phenyl-1H-indazol-3-yl)-urea | 1-Benzo[1,3]di oxol-5-isocyanate | 4.19/ 407 | 5,97 ppm (s, 2H) ; 6,81 ppm (dd,J = 2,5 - 8,5 Hz, 1H) ; 6,84 ppm (d,J = 8,5 Hz, 1H) ; 7,22 ppm (d,J = 2,5 Hz, 1H) ; de 7,35 à 7,50 ppm (m, 5H) ; 7,65 ppm (s, 1H) ; 8,05 ppm (s, 1H) ; 9,56 ppm (s, 1H) ; 9,6 ppm (s, 1H) ; 12,65 (sl, 1H) |
| B11 | 1-(6-chlore-5-phenyl-1H-indazol-3-yl)-3-(3,5-dimethyl-isoxazol-4-yl)-urea | 3,5-dimethyl-isoxazol-4-isocyanate | 3.76/ 382 | 2,13 ppm (s, 3H) ; 2,29 ppm (s, 3H) ; de 7,36 à 7,50 ppm (m, 5H) ; 7,64 ppm (s, 1H) ; 8,03 ppm (s, 1H) ; 8,75 ppm (s, 1H) ; 9,74 ppm (s, 1H) ; 12,68 ppm (sl, 1H) |
| B12 | 1-Benzyl-3-(6-chloro-5-phenyl-1H indazol-3-yl)-urea | Benzyle isocyanate | 4.2/ 377 | 4,43 ppm (d,J = 6 Hz, 2H) ; de 7,20 à 7,50 ppm (m, 10H) ; 7,58 ppm (s, 1H) ; 8,10 ppm (sl, 2H) ; 9,57 ppm (s, 1H) ; 12,50 ppm (s, 1H) |
| B13 | -(6-Chloro-5-phenyl-1H-indazol-3-yl)-3-phenethyl-thiourea | phenethyle thioiso-cyanate | | 2.96 ppm (t, J = 7.0 Hz, 2H) ; 3.86 ppm ( dt, J = 5.5 et 7.0 Hz, 2H) ; de 7.15 à 7.35 ppm (m, 5H) ; de 7.35 à 7.50 ppm ( m, 5H); 7.64 ppm, (s, 1H) ; 8.37 ppm ( s, 1H) ; 10.14 ppm (t, J = 5.5 Hz, 1H) ; 10.97 ppm (s, 1H) ; 12.73 ppm (s, 1H) |

### Exemple C1 : 1-(6-Chloro-5-phenyl-1H-indazol-3-yl)-3-[3-(4-methyl-piperazin-1-yl)-propyl]-urea

### Étape1

A 387.8 mg de 3-amino-5-phenyl-6-chloro-1-(2-triméthylsilyléthoxy)méthyl]-indazole dans 2 ml de chlorure de méthylène, sont ajoutés successivement 62µl de pyridine et 125µl de chloroformiate d'éthyle, Après 75 mn la réaction est terminée. Après hydrolyse, extraction et évaporation, on obtient 571 mg carbamate brut : de (6-Chloro-5-phenyl-1H-indazol-3-yl)-carbamic acid ethyle ester

### Etape2

A 106mg du carbamate précédent dans 2.5 ml de trifluorotoluene, on ajoute 377 mg de 3-aminopropyl-1-methyl piperazine et l'on conduit la réaction sous micro-ondes 20 mn à 200°C. Après purification par LC/MS préparative (acétonitrile/tampon pH=9) on obtient 60 mg de 1-[6-Chloro-5-phenyl-1-(2-trimethylsilanyl-ethoxymethyl)-1H-indazol-3-yl]-3-[3-(4-methyl-piperazin-1-yl)-propyl]-urea.

### Etape3

Le composé précédent est repris dans 2 ml d'un mélange 1/1 MeOH/HCl2N est porté au reflux 3h

RMN¹H [DMSO-d6] : 1,63(2H,m); 2,18(3H,s);2,33(10H,m);3,21(2H,m);7,36-7,48(5H,m);

7,58(1H,s);7,56(1H,t,J=5,5Hz);8,08(1H,s);9,37(1H,s)12,70(1H,s).

### Exemple : C₂ à C₅, C₇ à C₁₁, C₁₃ à C₁₈

Les produit C₂ à C₅, C₇ à C₁₁, C₁₃ à C₁₈ sont obtenus de manière équivalente au produit C1

| N° | Nom | Produit de départ | Temps de rétention/ [M+H]⁺ | RMN DMSO-d6 |
|---|---|---|---|---|
| C2 | 1-(6-Chloro-5-phenyl-1H-indazol-3-yl)-3-(3-imidazol-1-yl-propyl)-urea | 3-imidazol-propyl-1 amine | 3/ 395 | 2,05 ppm (m, 2H) ; 3,24 ppm (m, 2H) ; 4,25 ppm (t,J = 6 Hz, 2H) ; de 7,38 à 7,49 ppm (m, 5H) ; 7,61 ppm (s, 1H) ; 7,69 ppm (sl, 1H) ; 7,76 ppm (d,J = 5,5 Hz, 1H); 7,83 ppm (sl, 1H) ; 8,08 ppm (s, 1H) ; 9,19 ppm (s, 1H) ; 9,53 ppm (s, 1H) ; 12,53 ppm (sl, 1H) |
| C4 | 1-(6-Chloro-5-pheny1-1H-indazol-3-yl)-3-(2-hydroxy-ethyl)-urea | Ethanol-amine | 3.36/ 331 | 3,27 ppm (m, 2H) ; 3,49 ppm (t,J = 6,5 Hz, 2H) ; de 7,38 à 7,50 ppm (m, 5H) ; 7,59 ppm (s, 1H) ; 7,83 ppm (1, 1H) ; 8,10 ppm (s, 1H); 9,49 ppm (s, 1H); 12,50 ppm (sl, 1H) |
| C5 | 1-(6-Chloro-5-phenyl-1H-indazol-3-yl)-3-[3-(4-methyl-piperazin-1-yl)-propyl]-urea | (4-methyl-piperazin-1-yl)-propyl]-amine | 2.52/ 427 | |
| C7 | (6-Chloro-5-phenyl-1H-indazol-3-yl)-carbamic acid methyl ester | Chloroformiate de methyle | 4.1/ 302 | 3,66 ppm (s, 3H) ; de 7,33 ppm à 7,49 ppm (m, 5H); 7,65 ppm (s, 1H) ; 7,78 ppm, (s, 1H) ; 10,1 ppm (s, 1H) ; 12,80 ppm (s, 1H) |
| C8 | (6-Chloro-5-phenyl-1H-indazol-3-yl)-urea | Ammoniaque | 3.39/ 287 | 6,89 ppm (sl, 2H) ; de 7,37 ppm à 7,49 ppm (m, 5H) ; 7,59 ppm (s, 1H); 8,09 ppm (s, 1H) ; 9,37 ppm (s, 1H) ; 12,51 ppm (sl, 1H) |
| C9 | (6-Chloro-5-phenyl-1H-indazol-3-yl)-carbamic acid benzyl ester | Chloroformiate de benzyle | 4.5/ 378 | 5,14 ppm (s, 2H); de 7,29 à 7,49 ppm (m, 10H); 7,65 ppm (s, 1H) ; 7,76 ppm (s, 1H); 10,08 ppm (sl, 1H); 10,77 ppm (sl, 1H) |
| C10 | (6-Chloro-5-phenyl-1H indazol-3-yl)-carbamic acid allyl ester | Chloroformiate d'allyle | 4.4/ 328 | 4,61 ppm (dl,J=5Hz,2H); 5,21 ppm (dl,H= 11Hz, 1H) ; 5,34 ppm (dl,J = 17,5 Hz, 1H) ; 5,96 ppm (m, 1H) ; de 7,39 à 7,49 ppm (m, 5H) ; 7,65 ppm (s, 1H) ; 7,78 ppm (s, 1H); 10,06 ppm (sl, 1H) 12,76 ppm (sl, 1H) |
| C11 | (6-Chloro-5-phenyl-1H-indazol-3-yl)-carbamic acid isobutyl ester | Chloroformiate d'isobutyle | 4.55/ 344 | 0,90 ppm (d,J= 6,5 Hz, 6H) ; 1,90 ppm (m, 1H) ; 3,86 ppm (d,J=6,5 Hz 2H);de 7,38 ppm à 7,49 ppm (m, 5H); 7,66 ppm (s, 1H) ; 7,79 ppm (s, 1H) ; 9,93 ppm (sl, 1H); 12,93 ppm (sl, 1H) |
| C13 | 1-(3-Azetidin-1-yl-propyl)-3-(6-chloro-5-phenyl-1H-indazol-3-yl)-urea | Azetidine (double addition) | | 1,50 ppm (m, 2H) ; 2,02 ppm (m, 1H) ; 3,20 ppm (m, 2H) ; 2,54 ppm (masqué, 2H) ; 3,27 ppm (masqué, 4H) ; de 7,35 ppm à 7,50 ppm (m, 5H) ; 7,59 ppm (s, 1H) ; 7,72 ppm (tl,J = 6 Hz, 1H) ; 8,09 ppm (s, 1H) ; 9,44 ppm (s, 1H) ; 12,50 ppm (s, 1H) |
| C14 | 1-(6-chloro-5-phenyl-1H-indazol-3-yl)-3-(3-chloro-propyl)-urea | Azetidine (ouverture HCl) | 4.26/ 363 | 1,94 ppm (m, 2H) ; 3,30 ppm (masqué, 2H) ; 3,60 ppm (t,J = 6,5 Hz, 2H) ; de 7,38 ppm à 7,50 ppm (m, 5H); 7,58 ppm (s, 1H) ; 7,60 ppm (tl,J = 6 Hz, 1H) ; 8,07 ppm (s, 1H) ; 9,40 ppm (s, 1H) ; 12,41 ppm (s, 1H) |
| C15 | 1-(6,7-Difluoro-5-phenyl-1H-indazol-3-yl)-3-(3-imidazol-1-yl-propyl)-urea | 3-imidazol-1-yl-propyl amine | | 1.94 ppm (m, 2H) ; 3.18 ppm (q, J = 6.5 Hz, 2H) ; 4.02 ppm (t, J = 6.5 Hz, 2H) ; 6.89 ppm (s, 1H) ; 7.21 ppm (s, 1H) ; 7.42 ppm (tl, J = 7.5 Hz, 1H) ; 7.50 ppm (tl, J = 7.5 Hz, 2H) ; 7.55 ppm (dl, J = 7.5 Hz, 2H); 7.65 ppm ( s, 1H) ; 7.73 ppm (tl, J = 6.5 Hz, 1H) ; 8.05 ppm (d, J = 6.0 Hz, 1H) ; 9.58 ppm (s, 1H) |
| C16 | 1-(3-Amino-propyl)-3-(6-chloro-5-phenyl-1H-indazol-3-yl)-urea | 3-Amino-propyl amine | 2.74/ 344 | 1,77 ppm (m, 2H) ; 2,81 ppm (m, 2H) ; 3;28 ppm (m, 2H) ; de 7,38 ppm à 7,50 ppm (m, 5H) ; 7,60 ppm (s, 1H) ; 7,81 ppm (m, 3H); 8,08 ppm (s, 1H) ; 9,54 ppm (s, 1H) ; 12,54 pmpm (s, 1H) |
| C17 | 1-(6-Chloro-5-phenyl-1H-indazol-3-yl)-3-[4-(4-pyridin-3-yl-imidazol-1-yl)-butyl]-urea | 4-(4-pyridin-3-yl-imidazol-1-yl)-butyl amine | 2.95/ 486 | 1,51 ppm (m, 2H) ; 1,90 ppm (m, 2H) ; 3,26 ppm (m, 2H) ; 4,23 ppm (t,J = 7 Hz, 2H) ; de 7,37 ppm à 7,49 ppm (m, 5H) ; 7,58 ppm (s, 1H) ; 7,75 ppm (m, 2H) ; 8,08 ppm (s, 1H) ; 8,37 ppm (d,J = 2 Hz, 1H) ; 8,47 ppm (m, 1H) ; 8,69 ppm (dd,J = 1,5 - 5Hz, 1H) ; 9,00 ppm (sl, 1H) ; 9,12 ppm (d,J = 2 Hz, 1H) ; 9,48 ppm (s, 1H) ; 12,40 ppm (sl, 1H) |
| C18 | 1-(6-Chloro-5-phenyl-1H-indazol-3-yl)-3-(2-pyrrolidin-1-yl-ethyl)-urea | 2-pyrrolidin-1-yl-ethyl)-amine | 2.8/ 384 | 1,83 ppm (m, 2H) ; 1,99 ppm (m, 2H) ; 3,03 ppm (m, 2H) ; 3,28 ppm (m, 2H) ; 3,56 ppm (masqué, 4H) ; de 7,36 ppm à 7,49 ppm (m, 5H) ; 7,61 ppm (s, 1H) ; 7,80 ppm (tl,J = 5,5 Hz, 1H) ; 8,08 ppm (s, 1H) ; 9,65 ppm (s, 1H), 10,02 ppm (sl, |

### Exemple E1 : N-(6-chloro-5-phényl-1H-indazol-3-yl)-3-méthoxy benzènesulfonamide

*Etape* 1: 0.236cm³ de pyridine et 26.5 mg de chlorure de 3-méthoxyphényl sulfonyle sont additionnés à une solution de 54.1 mg de 3-amino-5-phenyl-6-chloro-1-(2-triméthylsilyléthoxy)méthyl]-indazole dans 2ml de chorure de méthylène. Le milieu réactionnel est agité pendant 24 heures à température ambiante puis évaporé. La purification du brut par chromatographie sur silice (éluant: chlorure de méthylène/acétone (98/2, v/v)) permet d'obtenir 70mg de 1N-[6-chloro-5-phényl-1-(2-triméthylsilanyl-éthoxyméthyl)-1H-indazol-3-yl]-3-méthoxy-benzènesulfonamide (mousse incolore).

Spectre de masse : 546[M+H]⁺; temps de rétention : 4.24 minutes.

RMN¹H [DMSO-d6] : 10.96 (1H, s) ; 7.37 (1H, s) ; 7.58 (1H, s) ; 7.30-7.55 (8H, m) ; 7.17 (1H, dd) ; 5.63 (2H, s) ; 3.74 (3H, s) ; 3.38 (2H, t, J=8Hz) ; 0.74 (2H, t, J=8Hz) ; -0.12 (9H, s).

*Etape 2 :* 1 cm³ de HCl 2N est additionné à une solution de 10.8mg de 1N-[6-chloro-5-phényl-1-(2-triméthylsilanyl-éthoxyméthyl)-1H-indazol-3-yl]-3-méthoxy-benzènesulfonamide dans 1cm³ de méthanol. Le milieu réactionnel est agité à température ambiante pendant 48 heures, 1 heure à reflux puis évaporé. Le solide obtenu est séché sous vide pour donner 8mg de N-(6-chloro-5-phényl-1H-indazol-3-yl)-3-méthoxy-benzènesulfonamide (solide incolore).

Spectre de masse : 414[M+H]⁺; temps de rétention : 4.04 minutes.

RMN¹H [DMSO-d6] : 12.90 (1H, s large) ; 10.74(1H, s large) ; 7.67 (1H, s) ; 7.31-7.56 (10H, s) ; 7.20 (1H, dd) ; 3.77 (3H, s).

Les compositions pharmaceutiques selon l'invention sont constitués par un composé de formule (I) ou un sel d'un tel composé, à l'état pur ou sous forme d'une composition dans laquelle il est associé à tout autre produit pharmaceutiquement compatible, pouvant être inerte ou physiologiquement actif. Les médicaments selon l'invention peuvent être employés par voie orale, parentérale, rectale ou topique.

Comme compositions solides pour administration orale, peuvent être utilisés des comprimés, des pilules, des poudres (capsules de gélatine, cachets) ou des granulés. Dans ces compositions, le principe actif selon l'invention est mélangé à un ou plusieurs diluants inertes, tels que amidon, cellulose, saccharose, lactose ou silice, sous courant d'argon. Ces compositions peuvent également comprendre des substances autres que les diluants, par exemple un ou plusieurs lubrifiants tels que le stéarate de magnésium ou le talc, un colorant, un enrobage (dragées) ou un vernis.

Comme compositions liquides pour administration orale, on peut utiliser des solutions, des suspensions, des émulsions, des sirops et élixirs pharmaceutiquement acceptables contenant des diluants inertes tels que l'eau, l'éthanol, le glycérol, les huiles végétales ou l'huile de paraffine. Ces compositions peuvent comprendre des substances autres que les diluants, par exemple des produits mouillants, édulcorants, épaississants, aromatisants ou stabilisants.

Les compositions stériles pour administration parentérale, peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou d'autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans de l'eau stérile ou tout autre milieu stérile injectable.

Les compositions pour administration rectale sont les suppositoires ou les capsules rectales qui contiennent, outre le produit actif, des excipients tels que le beurre de cacao, des glycérides semi-synthétiques ou des polyéthylèneglycols.

Les compositions pour administration topique peuvent être par exemple des crèmes, lotions, collyres, collutoires, gouttes nasales ou aérosols.

L'invention a pour objet les composés et leur utilisation d'aminoindazoles de formule (I) et leurs sels pharmaceutiquement acceptables pour la préparation de compositions pharmaceutiques destinées à prévenir et traiter les maladies pouvant résulter d'une activité anormale de kinases comme par exemple celles impliquées dans les maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, les maladies du métabolisme, le diabète de type II, l'hypertension essentielle, les maladies cardiovasculaires athérosclérotiques, le syndrome des ovaires polycystiques, le syndrome X, l'immunodéficience et le cancer,

Comme activité anormale de kinase on peut citer par exemple celle de la PI3K, AkT, GSK3béta, des CDK's ...

En thérapeutique humaine, les composés selon l'invention sont particulièrement utiles pour le traitement et/ou la prévention des maladies neurodégénératives, la maladie d'Alzheimer, de Parkinson, la démence frontopariétale, la dégénération corticobasale, la maladie de Pick, les accidents cérébrovasculaires, les traumatismes crâniens et spinaux et neuropathies périphériques, l'obésité, les maladies du métabolisme, le diabète de type II, l'hypertension essentielle, les maladies cardiovasculaires athérosclérotiques, le syndrome des ovaires polycystiques, le syndrome X, l'immunodéficience et le cancer.

Les doses dépendent de l'effet recherché, de la durée du traitement et de la voie d'administration utilisée; elles sont généralement comprises entre 5 mg et 1000 mg par jour par voie orale pour un adulte avec des doses unitaires allant de 1 mg à 250 mg de substance active.

D'une façon générale, le médecin déterminera la posologie appropriée en fonction de l'âge, du poids et de tous les autres facteurs propres au sujet à traiter.

Les exemples suivants illustrent des compositions selon invention :

### EXEMPLE A

On prépare, selon la technique habituelle, des gélules dosées à 50 mg de produit actif ayant la composition suivante :
- Composé de formule (I) 50 mg
- Cellulose 18 mg
- Lactose 55 mg
- Silice colloïdale 1 mg
- Carboxyméthylamidon sodique 10 mg
- Talc 10 mg
- Stéarate de magnésium 1 mg

### EXEMPLE B

On prépare selon la technique habituelle des comprimés dosés à 50 mg de produit actif ayant la composition suivante :
- Composé de formule (I) 50 mg
- Lactose 104 mg
- Cellulose 40 mg
- Polyvidone 10 mg
- Carboxyméthylamidon sodique 22 mg
- Talc 10 mg
- Stéarate de magnésium 2 mg
- Silice colloïdale 2 mg
- Mélange d'hydroxyméthylcellulose, glycérine, oxyde de titane (72-3,5-24,5) q.s.p. 1 comprimé pelliculé terminé à 245 mg

### EXEMPLE C

On prépare une solution injectable contenant 10 mg de produit actif ayant la composition suivante :
- Composé de formule (I) 10 mg
- Acide benzoïque 80 mg
- Alcool benzylique 0,06 ml
- Benzoate de sodium 80 mg
- Ethanol à 95 % 0,4 ml
- Hydroxyde de sodium 24 mg
- Propylène glycol 1,6 ml
- Eau q.s.p. 4 ml

## Revendications

1. Composés de formule (I) dans laquelle : 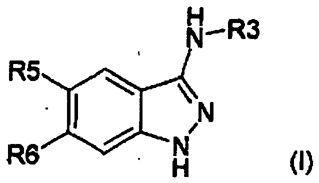
R3 est un radical (1-6C)alkyle, aryle, aryle(1-6C)alkyle, hétéroaryle, hétéroaryle(1-6C)alkyle, aryle ou hétéroaryle fusionné à un cycloalkyle (1-10C), hétérocycle, hétérocycloalkyle, cycloalkyle, adamantyle, polycycloalkyles, alkényle, alkynyle, CONR1R2, CSNR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, oxo, trifluorométhyle, trifluorométhylsulfanyle, trifluorométhoxy, (1-6C)alkyle ;
R5 est un aryle éventuellement substitué par 1 ou plusieurs substituants choisis parmi halogène, CN, NO₂, NH₂, OH, OR10, COOH, C(O)OR10, -O-C(O)R10, NR10RH, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifluorométhoxy, (1-6C)alkyle;
R6 est un radical halogène, méthyle, cyclopropyle, CN, OH, méthoxy, trifluorométhyle, éthylényle, acétylényle, trifluorométhoxy, N02, NH2, NMe2 ;
R1, R2, R₁₀ et R₁₁ sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy ;
leurs racémiques, énantiomères, diastéréoisomères et leurs mélanges, leurs tautomères et leurs sels pharmaceutiquement acceptables.

2. Composés de formule (I) selon la revendication 1 dans laquelle : 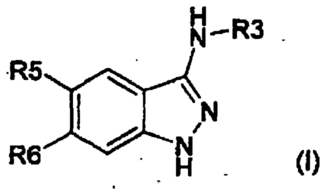
R3 est un radical (1-6C)alkyle, aryle, aryle(1-6C)alkyle, hétéroaryle, hétéroaryle(1-6C)alkyle, aryle ou hétéroaryle fusionné à un cycloalkyle (1-10C), héterocycle, hétérocycloalkyle, cycloalkyle, adamantyle, polycycloalkyles, alkényle, alkynyle, CONR1R2, CSNR1R2, COOR1, SO2R1, C(=NH)NR1 ; ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO2-O-R1, aryle, hétéroaryle, formyle, oxo, trifluorométhyle, trifluorométhylsulfanyle, trifluorométhoxy, (1-6C)alkyle
R5 est un phényl éventuellement substitués par 1 ou plusieurs substituants choisis parmi halogène, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryle, hétéroaryle, formyle, trifluorométhyle, trifluorométhoxy, (1-6C)alkyle ;
R6 est un radical halogène, méthyle, cyclopropyle, CN, OH, méthoxy, trifluorométhyle, éthylényle, acétylényle, trifluorométhoxy, N02, NH2, NMe2 ;
R1, R2, R10 et R11 sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, oxo, trifluorométhyle, trifluorométhoxy ;
leurs racémiques, énantiomères, diastéréoisomères et leurs mélanges, leurs tautomères et leurs sels pharmaceutiquement acceptables.

3. Composé selon la revendication 1 **caractérisé par le fait qu'**il est choisi parmi :
N-(bicyclo[2,2,1]hept-5-en-2ylmethyl)-6-chloro-5-phenyl-1H-indazol-3-amine
6-chloro-N-(3,3-dimethylbutyl)-5-phenyl-1H-indazol-3-amine
6-chloro-N-(3-phenylpropyl)-5-phenyl-1H-indazol-3-amine
6-chloro-N-(cyclopropylmethyl)-5-phenyl-1H-indazol-3-amine
6-chloro-N-(cyclopentylmethyl)-5-phenyl-1H-indazol-3-amine
6-chloro-N-[3-(methylthio)propyl]-5-phenyl-1H-indazol-3-amine
6-chloro-N-(phenylethyl)-5-phenyl-1H-indazol-3-amine
6-chloro-N-(cyclohexylmethyl)-5-phenyl-1H-indazol-3-amine
6-chloro-N-propyl-5-phenyl-1H-indazol-3-amine
6-chloro-N-(2,2,3,3,4,4,4-heptafluorobutyl)-5-phenyl-1H-indazol-3-amine, hydrate
5-chloro N-(4,4,4-trifluorobutyl)-5-phenyl-1H-indazol-3-amine
6-chloro-N-[(4-methoxyphenyl)methyl]-5-phenyl-1H-indazol-3-amine
6-chloro-N-(phenylmethyl)-5-phenyl-1H-indazol-3-amine
6-chloro-N-[(4-cyanophenyl)methyl]-5-pbenyl-1H-indazol-3-amine
N-[(4-chlorophenyl)methyl]-6-chloro-5-phenyl-1H-indazol-3-amine
6-chloro-N-[(3-methoxyphenyl)methyl]-5-phenyl-1H-indazol-3-amine
6-chloro-N-[[4-(trifluoromethoxy)phenyl]methyl]-5-phenyl-1H-indazol-3-amine
N-[4-[[[6-chloro-5-phenyl-1H-indazol-3-yl]amino]methy]phenyl]-acetamide
6-chloro-N-[(3,5-dichlorophenyl)methyl]-5-phenyl-1H-indazol-3-amine
6-chloro-5-phenyl N-[[4-(trifluoromethyl)phenyl]methyl]-1H-indazol-3-amine
6-chloro-N-[(4-fluorophenyl)methyl]-5-phenyl-1H-indazol-3-amine
6-chloro-N-[3-(4-methylphenoxy)phenylmethyl]-5-phenyl-1H-indazol-3-amine
N-(2,2,3,3,4,4,4-heptafluorobutyl]-6-chloro-5-phenyl-1H-indazol-3-amine
6-chloro-5-phenyl-N-[[3,5-bis(trifluoromethyl)phenyl]methyl]-1H-indazol-3-amine
6-chloro-5-phenyl-N-[[3-(trifluoromethyl)phényl]methyl]-1H-indazol-3-amine
6-chloro-N-[(6-methoxy-2-naphthalenyl)methyl]-5-phenyl-1H-indazol-3-amine
6-chloro-N-[(pentafluorophenyl)methyl]-5-phenyl-1H-indazol-3-amine
6-chloro-N-[[4-(methylthio)phenyl]methyl]-5-phenyl-1H-indazol-3-amine
N-[(4-chloro-3-fluorophenyl)methyl]-6-chloro-5-phenyl-1H-indazol-3-amine
6-chloro-5-phenyl-N-(3,3,3-trifluoropropyl)-1H-indazol-3-amine
6-chloro-5-phenyl-N-(3-thienylmethyl)-1H-indazol-3-amine
N-(bicyclo[2,2,1]hept-5-en-ylmethyl)-6-chloro-5-phenyl-1H-indazol-3-amine
N-([1,1'-biphenyl]-4-ylmethyl)-6-chloro-5-phenyl-1H-indazol-3-amine
5-chloro-N-[[4-(dimethylamino)phenyl]methy]-5-phenyl-1H-indazol-3-amine
N-([2,2'-bithiophen]-5-ylmethyl)-6-chloro-5-phenyl-1H-indazol-3-amine
6-chloro-5-phenyl-N-[[1-(phenylmethyl)-1H-imidazol-2-yl]methyl]-1H-indazol-3-amine
6-chloro-N-[[1-methyl-1H-imidazol-2-yl]methyl]-5-phenyl-1H-indazol-3-amine
6-chloro-N-[(1-methyl-1H-indol-3-yl)methyl]-5-phenyl-1H-indazol-3-amine
6-chloro-N-[(5-methyl-2-furanyl)methyl]-5-phenyl-1H-indazol-3-amine
6-chloro-5-phenyl-N-(1H-pyrrol-2-ylmethyl)-1H-indazol-3-amine
6-chloro-5-phenyl-N-(1H-imidazol-2-yl)methyl]-1H-indazol-3-amine
6-chloro-5-phenyl-N-(1H-imidazol-4-yl)methyl]-1H-indazol-3-amine
6-chloro-5-phenyl-N-(1H-pyrazol-3-ylmethyl)-1H-indazol-3-amine
6-chloro-N-[[2-methyl-1H-imidazol-4-yl]methyl]-5-phenyl-1H-indazol-3-amine
6-chloro-N-[(3,5-dimethyl-1-phenyl-1H-pyrazol-4-yl)methyl]-5-phenyl-1H-indazol-" 3,amine
6-chloro-5-phenyl-N-[12-phenyl-1H-imidazol-4-yl]methyl]-1H-indazol-3-amine
6-chloro-N-[[5-(4-chlorophenyl)-2-furanyl]methyl]-5-phenyl-1H-indazol-3-amine
6-chloro-5-phenyl-N-[(1-methyl-1H-pyrrol-2-yl)methyl]-1H-indazol-3-amine
4-[5-[[[6-chloro-5-phenyl-1H-imidazol-3-yl]amino]methyl]-2-furanyl]-Benzenesulfonamide
6-chloro-5-phenyl-N-(3-thienylmethyl)-1H-indazol-3-amine
6-chloro-5-phenyl-N-[[2-phenyl-1H-imidazol-4-yl]methyl]-1H-indazol-3-amine
2-[[[6-chloro-5-phenyl-1H-indazol-3-yl]amino]methyl]-5-(methylthio)-1H-imidazole-4-carboxylate d'éthyle
6-chloro-5-phenyl-N-[[5-[4-(trifluoromethyl)phenyl]-2-furanyl]methyl]-1H-indazol-3-amine
6-chloro-5-phenyl-N-[2-(1-piperidinyl)ethyl]-1H-indazol-3-amine
6-chloro-N-[2-(4-morpholinyl)ethyl]-5-phenyl-1H-indazol-3-amine
N-(6-chloro-5-phenyl-1H-indazol-3-yl)-N'-(3,5-dichlorophenyl)-Urée
N-(6-chloro-5-phenyl-1H-indazol-3-yl)-N'-(2-propenyl)- Urée
N-(6-chloro-5-phenyl-1H-indazol-3-yl)-N'-(phenylmethyl)- Urée
N-(6-chloro-5-phenyl-1H-indazol-3-yl)-N'-4-(phenoxyphenyl)- Urée
N-(6-chloro-5-phenyl-1H-indazol-3-yl)-N'-[(4-methoxyphenyl)methyl]- Urée.
N-(6-chloro-5-phenyl-1H-indazol-3-yl)-N'-[4-(trifluoromethyl)phenyl]-Urée
N-(6-chloro-5-phenyl-1H-indazol-3-yl)-N'-(4-methoxyphenyl)- Urée
N-(6-chloro-5-phenyl-1H-indazol-3-yl)-N'-cyclohexyl- Urée
N-(6-chloro-5-phenyl-1H-indazol-3-yl)-N'-propyl- Urée
N-(6-chloro-5-phenyl-1H-indazol-3-yl)-N'-(4-chlorophenyl)- Urée
N-(6-chloro-5-phenyl-1H-indazol-3-yl)-N'-(4-fluorophenyl)- Urée
N-[6-chloro-5-phenyl-1H-indazol-3-yl]-N'-tricyclo[3.3.1.13,7]dec-1-yl- Urée
N-(6-chloro-5-phenyl-1H-indazol-3-yl)-N'-(4-methylphenyl)- Urée
N-[6-chloro-5-phenyl-1H-indazol-3-yl]-4-methyl-Benzenesulfonamide
N-[6-chloro-5-phenyl-1H-indazol-3-yl]-Methanesulfonamide
N-[6-chloro-5-phenyl-1H-indazol-3-yl]-2-propanesulfonamide
N-[6-chloro-5-phenyl-1H-indazol-3-yl]-2,2,2-trifluoro-ethanesulfonamide
N-[6-chloro-5-phenyl-1H-indazol-3-yl]-2-thiophenesulfonamide
N-[6-chloro-5-phenyl-1H-indazol-3-yl]-Benzenesulfonamide
N-[6-chloro-5-phenyl-1H-indazol-3-yl]-4-(trifluoromethyl)-Benzenesulfonamide
N-[6-cHoro-5-phenyl-1H-indazol-3-yl]-5-(3-isoxazoyl)-2-thiophenesulfonamide
N-[6-chloro-5-phenyl-1H-indazol-3-yl]-4-fluoro-Benzenesulfonamide
N-[6-ohloro-5-phenyl-1H-indazol-3-yl]-4-methoxy-Benzenesulfonamide
N-6-chloro-5-phenyl-1H-indazol-3-yl]-Benzenemethanesulfonamide
N-[6-chloro-5-phenyl-1H-indazol-3-yl]-1-mehyl-1H-imidazole-4-sulfonamide
N-[6-chloro-5-phenyl-1H-indazol-3-yl]-4-(1,1-dimethylethyl)-Benzenesulfonamide
N-[4-[[(6-chloro-5-phenyl-1H-indazol-3-yl)amino]sulfonyl]phenyl]-Acetamide
N-[6-chloro-5-phenyl-1H-indazol-3-yl]-4-methyl-Benzenemethanesulfonamide
6-chloro-N-(pentafluorophenyl)-5-phenyl-1H-indazol-3-amine
6-chloro-N-(3,4-difluorophenyl)-5-phenyl-1H-indazol-3-amine
6-chloro-5-phenyl-N-(2,3,5,6-tetrafluorophenyl)-1H-indazol-3-amine
6-chloro-5-phenyl-N-(2,4,6-trifluorophenyl)-1H-indazol-3-amine
6-chloro-N-(4-fluoorophenyl)-5-phenyl-1H-indazol-3-amine
6-chloro-N-[3-(trifluoromethyl)phenyl]-5-phenyl-1H-indazol-3-amino
6-chloro-N-[4-(trifluoromethyl)phenyl]-5-phenyl-1H-indazol-3-amine
6-chloro-N-[3-fluoro-5-(trifluoromethyl)phenyl]-5-phenyl-1H-indazol-3-amine
6-chloro-N-(4-nitrophenyl)-5-phenyl-1H-indazol-3-amine
6-chloro-N-(3-nitrophenyl)-5-phenyl-1H-indazol-3-amine
6-chloro-N-(3-methoxyphenyl)-5-phenyl-1H-indazol-3-amine
6-chloro-N-(4-methoxyphenyl)-5-phenyl-1H-indazol-3-amine
6-chloro-N,5-diphenyl-1H-indazol-3-amine
6-chloro-N-(1-pyridinyl)-5-phenyl-1H-indazol-3-amine
6-chloro-N-(2-pyridinyl)-5-phenyl-1H-indazol-3-amine
leur racémiques, énantiomères, diastéréoisomères et leurs mélanges, leurs tautomères et leurs sels pharmaceutiquement acceptables.

4. Composé selon l'une quelconque des revendications 1 à 2 **caractérisé par le fait qu'**il est choisi parmi :
N-butyl-6-chloro-5-phenyl-1H-indazol-3-amine
3-(6-Chloro-5-phenyl-1H-indazol-3-ylamino)-thiophene-2-carbonitrile
(6-Chloro-5-phenyl-1H-indazol-3-yl)-pyridin-2-yl-amine
(6-Chloro-5-phenyl-1H-indazol-3-yl)-(5-nitro-pyridin-2-yl)-amine
(6-Chloro-5-phenyl-1H-indazol-3-yl)-(6-methoxy-pyridin-2-yl)-amine
N-(6-chloro-5-phenyl-1H-indazol-3-yl)-N'-phenyl- Urée
1-(6-Chloro-5-phenyl-1H-indazol-3-yl)-3-(4-ethoxy-phenyl)-urea
1-(6-Chloro-5-phenyl-1H-indazol-3-yl)-3-(3,4-dichloro-phenyl)-urea
3-[3-(6-Chloro-S-phenyl-1H-indazol-3-yl)-ureido]-propionic acid methyl ester
1-(6-Chloro-5-phenyl-1H-indazol-3-yl)-3-(4-dimethylamino-phenyl)-urea
1-(6-Chloro-5-phenyl-1H-indazol-3-yl)-3-isopropyl-urea
1-(6-Chloro-5-phenyl-1H-indazol-3-yl)-3-cyclohexyl-urea
1-(6-Chloro-5-phenyl-1H-indazol-3-yl)-3-(3-trifluoromethyl-phenyl)-urea
1-(6-Chloro-5-phenyl-1H-indazol-3-yl)-3-(2-thiophen-2-yl-ethyl)-urea
1-Benzo[1,3]dioxol-5-yl-3-(6-chloro-5-phenyl-1H-indazol-3-yl)-urea
1-(6-Chloro-5-phenyl-1H-indazol-3-yl)-3-(3,5-dimethyl-isoxazol-4-yl)-urea
1-Benzyl-3-(6-chloro-5-phenyl-1H-indazol-3-yl)-urea
1-(6-Chloro-5-phenyl-1H-indazol-3-yl)-3-phenethyl-thiourea
1-(6-Chloro-5-phenyl-1H-indazol-3-yl)-3-[3-(4-methyl-piperazin-1-yl)-propyl]-urea
1-(6-Chloro-5-phenyl-1H-indazol-3-yl)-3-(3-imidazol-1-yl-propyl)-urea
1-(6-Chloro-5-phenyl-1H-indazol-3-yl)-3-(2-hydroxy-ethyl)-urea
1-(6-Chloro-5-phenyl-1H-indazol-3-yl)-3-[3-(4-methyl-piperazin-1-yl)-propyl]-urea
(6-Chloro-5-phenyl-1H-indazol-3-yl)-carbamic acid methyl ester
(6-Chloro-5-phenyl-1H-indazol-3-yl)-urea
(6-Chloro-5-phenyl-1H-indazol-3-yl)-carbamic acid benzyl ester
(6-Chloro-5-phenyl-1H-indazol-3-yl)-carbamic acid allyl ester
(6-Chloro-5-phenyl-1H-indazol-3-yl)-carbamic acid isobutyl ester
1-(3-Azetidin-1-yl-propyl)-3-(6-chloro-5-phenyl-1H-indazol-3-yl)-urea
1-(6-Chloro-5-phenyl-1H-indazol-3-yl)-3-(3-chloro-propyl)-urea
1-(6,7-Difluoro-5-phenyl-1H-indazol-3-yl)-3-(3-imidazol-1-yl-propyl)-urea
1-(3-Amino-propyl)-3-(6-chloro-5-phenyl-1H-indazol-3-yl)-urea
1-(6-Chloro-5-phenyl-1H-indazol-3-yl)-3-[4-(4-pyridin-3-yl-imidazol-1-yl)-butyl]-urea
1-(6-Chloro-5-phenyl-1H-indazol-3-yl)-3-(2-pyrrolidin-1-yl-ethyl)-urea
N-(6-chloro-5-phényl-1H-indazol-3-yl)-3-méthoxy-benzènesulfonamide
son racémique, ses énantiomères, tautomères ainsi que leurs sels pharmaceutiquement acceptables.

5. Composé selon l'une quelconque des revendications 1 à 4 utilisé pour préparer un médicament.

6. Composition pharmaceutique **caractérisée par le fait qu'**elle comprend dans un milieu pharmaceutiquement acceptable, un composé défini selon l'une quelconque des revendications 1 à 4.

7. Médicament, qui comprend, dans un milieu pharmaceutiquement. acceptable,au moins un composé défini selon l'une quelconque des revendications 1 à 4 pour son application thérapeutique dans le traitement des maladies dans lesquelles une phosphorylation de la protéine Tau est observée.

8. Médicament selon la revendication 7 **caractérisé par le fait qu'**il contient au moins un composé défini selon l'une quelconque des revendications 1 à 4 pour son application thérapeutique dans le traitement des maladies neurodégénératives, les accidents cérébrovasculaires, les traumatismes crâniens et spinaux et neuropathies périphériques, l'obésité, les maladies du métabolisme, le diabètede type II, l'hypertension essentielle, les maladies cardiovasculaires athérosclérotiques, le syndrome des ovaires polycystiques, le syndrome X, l'imunodéficience et le cancer.

9. Médicament selon la revendication 8 **caractérisé par le fait que** la maladie neurodégérative est soit la maladie d'Alzheimer, de Parkinson, la démence frontopariétale, la dégénération corticobasale ou la maladie de Pick.

10. Procédé de préparation des composés de formule (I) tels que définis dans la revendication 1 et pour lequel R3 est (1-6C)alkyle, aryle(1-6C)alkyle, hétéroaryle(1-6C)alkyle, hétérocycloalkyle, cycloalkyle, polycycloakyles, ces radicaux étant éventuellement substitués par 1 ou plusieurs substituants choisis parmi halogène, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryle, hétéroaryle, formyle, trifluorométhyle, trifluorométhylsulfanyle, trifluorométhoxy, (1-6C)alkyle et R1, R2, sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy ; à partir d'un dérive de formule (I) où R3 est H, d'un dérivé R1CHO et du triacétoxyborohydrure de sodium dans du dichlorométhane et transforme éventuellement le produit obtenu en sel pharmaceutiquement acceptable.

11. Procédé de préparation des composés de formule (I) tels que définis dans la revendication 1 et pour lequel R3 est CONR1R2, R1, R2, sont indépendamment l'un de l'autre un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy à partir de OCNR1 et d'un dérivé de formule (I) où R3 est H dans du tétrahydrofurane et transforme éventuellement le produit obtenu en sel pharmaceutiquement acceptable.

12. Procédé de préparation des composés de formule (I) tels que définis dans la revendication 1 et pour lequel R3 est SO2R1 et R1 est un hydrogène, (1-6C)alkyle, aryle, alkényle, alkynyle, hétéroaryle étant eux mêmes éventuellement substitués par 1 ou plusieurs substituants choisis parmi halogène, (1-6C)alkyle, (1-6C)alcoxy, CN, NO₂, NH₂, OH, COOH, COOalkyle, CONH₂, formyle, trifluorométhyle, trifluorométhoxy à partir de d'un chlorure de sulfonyle R1SO2C1 et d'un dérivé de formule (I) où R3 est H dans du dichlorométhane en présence d'une base et transforme éventuellement le composé obtenu en sel pharmaceutiquement acceptable.

13. A titre de produit intermédiaire 3-amino-5-phenyl-6-chloro-1-(2-triméthylsilyléthoxy)méthyl]-indazole.

## Claims

1. Compounds of formula (I), in which: 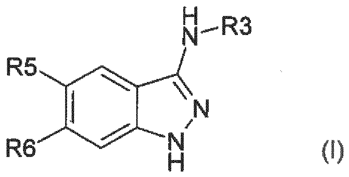
R3 is a (1-6C)alkyl, aryl, aryl(1-6C)alkyl, heteroaryl, heteroaryl(1-6C)alkyl, aryl or heteroaryl fused to a (1-10C) cycloalkyl, heterocycle, heterocycloalkyl, cycloalkyl, adamantyl, polycycloalkyl, alkenyl, alkynyl, CONR1R2, CSNR1R2, COOR1, SO₂R1 or C(=NH)NR1 radical; these radicals being optionally substituted with one or more substituents chosen from halogen, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryl, heteroaryl, formyl, oxo, trifluoromethyl, trifluoromethylsulfanyl, trifluoromethoxy and (1-6C)alkyl;
R5 is an aryl optionally substituted with one or more substituents chosen from halogen, CN, NO₂, NH₂, OH, OR10, COOH, C(O)OR10, -O-C(O)R10, NR10R11, NHC(O)R10, C(O)NR10R11, NHC(S)R10, C(S)NR10R11, SR10, S(O)R10, SO₂R10, NHSO₂R10, SO₂NR10R11, -O-SO₂R10, -SO₂-O-R10, aryl, heteroaryl, formyl, trifluoromethyl, trifluoromethoxy and (1-6C)alkyl;
R6 is a halogen, methyl, cyclopropyl, CN, OH, methoxy, trifluoromethyl, ethylenyl, acetylenyl, trifluoromethoxy, NO₂, NH₂ or NMe₂ radical;
R1 R2, R10 and R11 are, independently of each other, a hydrogen, (1-6C)alkyl, aryl, alkenyl, alkynyl or heteroaryl, which are themselves optionally substituted with one or more substituents chosen from halogen, (1-6C)alkyl, (1-6C)alkoxy, CN, NO₂, NH_{2,} OH, COOH, COOalkyl, CONH₂, formyl, oxo, trifluoromethyl and trifluoromethoxy;
racemates, enantiomers and diastereoisomers thereof and mixtures thereof, tautomers thereof and pharmaceutically acceptable salt thereof.

2. Compounds of formula (I) according to Claim 1, in which: 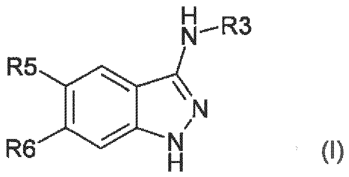
R3 is a (1-6C)alkyl, aryl, aryl(1-6C)alkyl, heteroaryl, heteroaryl(1-6C)alkyl, aryl or heteroaryl fused to a (1-10C) cycloalkyl, heterocycle, heterocycloalkyl, cycloalkyl, adamantyl, polycycloalkyl, alkenyl, alkynyl, CONR1R2, CSNR1R2, COOR1, SO₂R1 or C(=NH)NR1 radical; these radicals being optionally substituted with one or more substituents chosen from halogen, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryl, heteroaryl, formyl, oxo, trifluoromethyl, trifluoromethylsulfanyl, trifluoromethoxy and (1-6C)alkyl;
R5 is a phenyl optionally substituted with one or more substituents chosen from halogen, CN, NO₂, NH₂, OH, OR10, COOH, C(O)OR1O, -O-C(O)R10, NR10R11, NHC(O)R10, C(O)NR10R11, NHC(S)R10, C(S)NR10R11, SR10, S(O)R10,
SO₂R10, NHSO₂R10, SO₂NR10OR11, -O-SO₂R10, -SO₂-O-R10, aryl, heteroaryl, formyl, trifluoromethyl, trifluoromethoxy and (1-6C)alkyl;
R6 is a halogen, methyl, cyclopropyl, CN, OH, methoxy, trifluoromethyl, ethylenyl; acetylenyl, trifluoromethoxy, NO₂, NH₂ or NMe₂ radical;
R1, R2, R10 and R11 are, independently of each other, a hydrogen, (1-6C)alkyl, aryl, alkenyl, alkynyl or heteroaryl, which are themselves optionally substituted with one or more substituents chosen from halogen, (1-6C) alkyl, (1-6C)alkoxy, CN, NO₂, NH₂, OH, COOH, COOalkyl, CONH₂, formyl, oxo, trifluoromethyl, and trifluoromethoxy;
racemates, enantiomers and diastereoisomers thereof and mixtures thereof, tautomers thereof and pharmaceutically acceptable salt thereof.

3. Compound according to Claim 1, **characterized in that** it is chosen from:
N-(bicyclo[2.2.1]hept-5-en-2-ylmethyl)-6-chloro-5-enyl-1H-indazol-3-amine;
6-chloro-N-(3,3-dimethylbutyl)-5-phenyl-1H-indazol-3-amine;
6-chloro-N-(3-phenylpropyl)-5-phenyl-1H-indazol-3-amine;
6-chloro-N-(cyclopropylmethyl)-5-phenyl-1H-indazol-3-amine;
6-chloro-N-(cyclopentylmethyl)-5-phenyl-1H-indazol-3-amine;
6-chloro-N-[3-(methylthio)propyl]-5-phenyl-1H-indazol-3-amine;
6-chloro-N-(phenylethyl)-5-phenyl-1H-indazol-3-amine;
6-chloro-N-(cyclohexylmethyl)-5-phenyl-1H-indazol-3-amine;
6-chloro-N-propyl-5-phenyl-1H-indazol-3-amine;
6-chloro-N-(2,2,3,3,4,4,4-heptafluorobutyl)-5-phenyl-1H-indazol-3-amine hydrate;
6-chloro-N-(4,4,4-trifluorobutyl)-5-phenyl-1H-indazol-3-amine;
6-chloro-N-[(4-methoxyphenyl)methyl]-5-phenyl-1H-indazol-3-amine;
6-chloro-N-(phenylmethyl)-5-phenyl-1H-indazol-3-amine;
6-chloro-N-[(4-cyanophenyl)methyl]-5-phenyl-1H-indazol-3-amine;
N-[(4-chlorophenyl)methyl]-6-chloro-5-phenyl-1H-indazol-3-amine;
6-chloro-N-[(3-methoxyphenyl)methyl]-5-phenyl-1H-indazol-3-amine;
6-chloro-N-[[4-(trifluoromethoxy)phenyl]methyl]-5-phenyl-1H-indazol-3-amine;
N-[4-[[[6-chloro-5-phenyl-1H-indazol-3-yl]amino]-methyl]phenyl]acetamide;
6-chloro-N-[(3,5-dichlorophenyl)methyl]-5-phenyl-1H-indazol-3-amine;
6-chloro-5-phenyl-N-[[4-(trifluoromethyl)phenyl]-methyl]-1H-indazol-3-amine;
6-chloro-N-[(4-fluorophenyl)methyl]-5-phenyl-1H-indazol-3-amine;
6-chloro-N-[3-(4-methylphenoxy)phenylmethyl]-5-phenyl-1H-indazol-3-amine;
N-(2,2,3,3,4,4,4-heptafluorobutyl)-6-chloro-5-phenyl-1H-indazol-3-amine;
6-chloro-5-phenyl-N-[[3,5-bis(trifluoromethyl)phenyl]-methyl]-1H-indazol-3-amine;
6-chloro-5-phenyl-N-[[3-(trifluoromethyl)phenyl]-methyl]-1H-indazol-3-amine;
6-chloro-N-[(6-methoxy-2-naphthyl)methyl]-5-phenyl-1H-indazol-3-amine;
6-chloro-N-[(pentafluorophenyl)methyl]-5-phenyl-1H-indazol-3-amine;
6-chloro-N-[[4-(methylthio)phenyl]methyl]-5-phenyl-1H-indazol-3-amine;
N-[(4-chloro-3-fluorophenyl)methyl]-6-chloro-5-phenyl-1H-indazol-3-amine;
6-chloro-5-phenyl-N-(3,3,3-trifluoropropyl)-1H-indazol-3-amine;
6-chloro-5-phenyl-N-(3-thienylmethyl)-1-indazol-3-amine;
N-(bicyclo[2.2.1]hept-5-en-2-ylmethyl)-6-chloro-5-phenyl-1H-indazol-3-amine;
N-(1,1'-biphenyl-4-ylmethyl)-6-chloro-5-phenyl-1H-indazol-3-amine;
6-chloro-N-[[4-(dimethylamino)phenyl]methyl]-5-phenyl-1H-indazol-3-amine;
N-(2,2'-bithiophen-5-ylmethyl)-6-chloro-5-phenyl-1H-indazol-3-amine;
6-chloro-5-phenyl-N-[[1-(phenylmethyl)-1H-imidazol-2-yl]methyl]-1H-indazol-3-amine;
6-chloro-N-[[1-methyl-1H-imidazol-2-yl]methyl]-5-phenyl-1H-indazol-3-amine;
6-chloro-N-[(1-methyl-1H-indazol-3-yl)methyl]-5-phenyl-1H-indazol-3-amine;
6-chloro-N-[(5-methyl-2-furanyl)methyl]-5-phenyl-1H-indazol-3-amine;
6-chloro-5-phenyl-N-(1H-pyrrol-2-ylmethyl)-1H-indazol-3-amine;
6-chloro-5-phenyl-N-[(1H-imidazol-2-yl)methyl]-1H-indazol-3-amine;
6-chloro-5-phenyl-N-[(1H-imidazol-4-yl)methyl]-1H-indazol-3-amine;
6-chloro-5-phenyl-N-[(1H-pyrazol-3-ylmethyl)-1H-indazol-3-amine;
6-chloro-N-[[2-methyl-1H-imidazol-4-yl]methyl]-5-phenyl-1H-indazol-3-amine;
6-chloro-N-[(3,5-dimethyl-1-phenyl-1H-pyrazol-4-yl)methyl]-5-phenyl-1H-indazol-3-amine;
6-chloro-5-phenyl-N-[[2-phenyl-1H-imidazol-4-yl]methyl]-1H-indazol-3-amine;
6-chloro-N-[[5-(4-chlorophenyl)-2-furanyl]methyl]-5-phenyl-1H-indazol-3-amine;
6-chloro-5-phenyl-N-[(1-methyl-1H-pyrrol-2-yl)methyl] 1H-indazol-3-amine;
4-[5-[[[6-chloro-5-phenyl-1H-indazol-3-yl]amino] methyl]-2-furanyl]benzenesulfonamide;
6-chloro-5-phenyl-N-(3-thienylmethyl)-1H-indazol-3-amine;
6-chloro-5-phenyl-N-[[2-phenyl-1H-imidazol-4-yl]methyl]-1H-indazol-3-amine;
ethyl 2-[[[6-chloro-5-phenyl-1H-indazol-3-yl]amino]-methyl]-5-(methylthio)-1H-imidazol-4-carboxylate;
6-chloro-5-phenyl-N-[[5-[4-(trifluoromethyl)phenyl]-2-furanyl]methyl]-1H-indazol-3-amine;
6-chloro-5-phenyl-N-[2-(1-piperidinyl)ethyl]-1H-indazol-3-amine;
6-chloro-N-[2-(4-morpholinyl)ethyl]-5-phenyl-1H-indazol-3-amine;
N-(6-chloro-5-phenyl-1H-indazol-3-yl)-N'-(3,5-dichlorophenyl)urea;
N-(6-chloro-5-phenyl-1H-indazol-3-yl*)*-N'-(2-propenyl) urea;
N-(6-chloro-5-phenyl-1H-indazol-3-yl)-N'-(phenylmethyl)urea;
N-(6-chloro-5-phenyl-1H-indazol-3-yl)-N'-(4-phenoxyphenyl)urea;
N-(6-chloro-5-phenyl-1H-indazol-3-yl)-N'-(4-methoxyphenyl)methyl]urea;
N-(6-chloro-5-phenyl-1H-indazol-3-yl)-N'-[4-(trifluoromethyl)phenyl]urea;
N-(6-chloro-5-phenyl-1H-indazol-3-yl)-N'-(4-methoxyphenyl)urea;
N-(6-chloro-5-phenyl-1H-indazol-3-yl)-N'-cyclohexylurea;
N-(6-chloro-5-phenyl-1H-indazol-3-yl)-N'-propylurea;
N-(6-chloro-5-phenyl-1H-indazol-3-yl)-N'-(4-chlorophenyl)urea;
N-(6-chloro-5-phenyl-1H-indazol-3-yl)-N'-(4-fluorophenyl)urea;
N-[6-chloro-5-phenyl-1H-indazol-3-yl]-N'-(tricyclo[3.3.1.1^{3,7}]dec)-1-ylurea;
N-(6-chloro-5-phenyl-1H-indazol-3-yl)-N'-(4-methylphenyl)urea;
N-[6-chloro-5-phenyl-1H-indazol-3-yl]-4-methyl-benzenesulfonamide;
N-[6-chloro-5-phenyl-1H-indazol-3-yl]methanesulfon-amide;
N-[6-chloro-5-phenyl-1H-indazol-3-yl]-2-propanesulfonamide;
N-[6-chloro-5-phenyl-1H-indazol-3-yl]-2,2,2-trifluoroethanesulfonamide;
N-[6-chloro-5-phenyl-1H-indazol-3-yl]-2-thiophenesulfonamide;
N-[6-chloro-5-phenyl-1H-indazol-3-yl]benzenesulfonamide;
N-[6-chloro-5-phenyl-1H-indazol-3-yl]-4-(trifluoromethyl)benzenesulfonamide;
N-[6-chloro-5-phenyl-1H-indazol-3-yl]-5-(3-isoxazolyl)-2-thiophenesulfonamide;
N-[6-chloro-5-phenyl-1H-indazol-3-yl]-4-fluorobenzenesulfonamide;
N-[6-chloro-5-phenyl-1H-indazol-3-yl]-4-methoxybenzenesulfonamide;
N-[6-chloro-5-phenyl-1H-indazol-3-yl]benzenemethanesulfonamide;
N-[6-chloro-5-phenyl-1H-indazol-3-yl]-1-methyl-1H-imidazole-4-sulfonamide;
N-[6-chloro-5-phenyl-1H-indazol-3-yl]-4-(1,1-dimethylethyl)benzenesulfonamide;
N-[4-[[(6-chloro-5-phenyl-1H-indazol-3-yl)amino]sulfonyl]phenyl]acetamide;
N-[6-chloro-5-phenyl-1H-indazol-3-yl]-4-methylbenzenemethanesulfonamide;
6-chloro-N-(pentafluorophenyl)-5-phenyl-1H-indazol-3-amine;
6-chloro-N-(3,4-difluorophenyl)-5-phenyl-1H-indazol-3-amine;
6-chloro-5-phenyl-N-(2,3,5,6-tetrafluorophenyl)-1H-indazol-3-amine;
6-chloro-5-phenyl-N-(2,4,6-trifluorophenyl)-1H-indazol-3-amine;
6-chloro-N-(4-fluorophenyl)-5-phenyl-1H-indazol-3-amine;
6-chloro-N-[3-(trifluoromethyl)phenyl]-5-phenyl-1H-indazol-3-amine;
6-chloro-N-[4-(trifluoromethyl)phenyl]-5-phenyl-1H-indazol-3-amine;
6-chloro-N-[3-fluoro-5-(trifluoromethyl)phenyl]-5-phenyl-1H-indazol-3-amine;
6-chloro-N-(4-nitrophenyl)-5-phenyl-1H-indazol-3-amine;
6-chloro-N-(3-nitrophenyl)-5-phenyl-1H-indazol-3-amine;
6-chloro-N-(3-methoxyphenyl)-5-phenyl-1H-indazol-3-amino;
6-chloro-N-(4-methoxyphenyl)-5-phenyl-1H-indazol-3-amine;
6-chloro-N,5-diphenyl-1H-indazol-3-amine;
6-chloro-N-(1-pyridinyl)-5-phenyl-1H-indazol-3-amine;
6-chloro-N-(2-pyridinyl)-5-phenyl-1H-indazol-3-amine;
racemates, enantiomers and diastereoisomers thereof and
mixtures thereof, tautomers thereof and
pharmaceutically acceptable salt thereof.

4. Compound according to either of Claims 1 and 2, **characterized in that** it is chosen from:
N-butyl-6-chloro-5-phenyl-1H-indazol-3-amine;
3-(6-chloro-5-phenyl-1H-indazol-3-ylamino)thiophene-2-carbonitrile;
(6-chloro-5-phenyl-1H-indazol-3-yl) (pyridin-2-yl)amine;
(6-chloro-5-phenyl-1H-indazol-3-yl)(5-nitropyridin-2-yl)amine;
(6-chloro-5-phenyl-1H-indazol-3-yl)(6-methoxypyridin-2-yl)amine;
N-(6-chloro-5-phenyl-1H-indazol-3-yl)-N'-phenylurea;
1-(6-chloro-5-phenyl-1H-indazol-3-yl)-3-(4-ethoxyphenyl)urea;
1-(6-chloro-5-phenyl-1H-indazol-3-yl)-3-(3,4-dichlorophenyl)urea;
3[-(3-(6-chloro-5-phenyl-1H-indazol-3-yl)ureido]propionic acid methyl ester;
1-(6-chloro-5-phenyl-1H-indazol-3-yl)-3-(4-dimethylamino)phenyl)urea;
1-(6-chloro-5-phenyl-1H-indazol-3-yl)-3-isopropylurea;
1-(6-chloro-5-phenyl-1H-indazol-3-yl)-3-cyclohexylurea;
1-(6-chloro-5-phenyl-1H-indazol-3-yl)-3-(3-(trifluoromethyl)phenyl)urea;
1-(6-chloro-5-phenyl-1H-indazol-3-yl)-3-(2-(thiophen-2-yl)ethyl)urea;
1-(1,3-benzodioxol-5-yl)-3-(6-chloro-5-phenyl-1H-indazol-3-yl)urea;
1-(6-chloro-5-phenyl-1H-indazol-3-yl)-3-(3,5-dimethylisoxazol-4-yl)urea;
1-benzyl-3-(6-chloro-5-phenyl-1H-indazol-3-yl)urea;
1-(6-chloro-5-phenyl-1H-indazol-3-yl)-3-(phenethyl)thiourea;
1-(6-chloro-5-phenyl-1H-indazol-3-yl)-3-[3-(4-methylpiperazin-1-yl)propyl]urea;
1-(6-chloro-5-phenyl-1H-indazol-3-yl)-3-(3-imidazol-1-yl)propyl)urea;
1-(6-chloro-5-phenyl-1H-indazol-3-yl)-3-(2-ydroxyetyl)urea;
1-(6-chloro-5-phenyl-1H-indazol-3-yl)-3-[3-(4-methylpiperazin-1-yl)propyl]urea;
(6-chloro-5-phenyl-1H-indazol-3-yl)carbamic acid methyl ester;
(6-chloro-5-phenyl-1H-indazol-3-yl)urea;
(6-chloro-5-phenyl-1H-indazol-3-yl)carbamic acid benzyl ester;
(6-chloro-5-phenyl-1-indazol-3-yl)carbamic acid allyl ester;
(6-chloro-5-phenyl-1H-indazol-3-yl)carbamic acid isobutyl ester;
1-(3-(azetidin-1-yl)propyl)-3-((6-chloro-5-phenyl-1H-indazol-3-yl)urea;
1-(6-chloro-5-phenyl-1H-indazol-3-yl)-3-(3-chloropropyl)urea;
1-(6,7-difluoro-5-phenyl-1H-indazol-3-yl)-3-(3-imidazol-1-yl)propyl)urea;
1-(3-(aminopropyl)-3-(6-chloro-5-phenyl-1-indazol-3-yl)urea;
1-(6-chloro-5-phenyl-1H-indazol-3-yl)-3-[4-(4-pyridin-3-yl)imidazol-1-yl)butyl]urea;
1-(6-chloro-5-phenyl-1H-indazol-3-yl)-3-(2-pyrrolidin-1-ylethyl)urea;
N-(6-chloro-5-phenyl-1H-indazol-3-yl)-3-methoxybenzene-sulfonamide;
a racemate thereof, enantiomers and tautomers thereof and pharmaceutically acceptable salts thereof.

5. Compound according to any one of Claims 1 to 4, used for preparing a medicament.

6. Pharmaceutical composition, **characterized in that** it comprises, in a pharmaceutically acceptable medium, a compound defined according to any one of Claims 1 to 4.

7. Medicament, which comprises, in a pharmaceutically acceptable medium, at least one compound defined according to any one of Claims 1 to 4 for its therapeutic application in the treatment of diseases in which phosphorylation of the Tau protein is observed.

8. Medicament according to Claim 7, **characterized in that** it contains at least one compound defined according to any one of Claims 1 to 4, for its therapeutic application in the treatment of neurodegenerative diseases, strokes, cranial and spinal trauma and peripheral neuropathies, obesity, metabolic diseases, type II diabetes, essential hypertension, atherosclerotic cardiovascular diseases, polycystic ovary syndrome, syndrome X, immunodeficiency and cancer.

9. Medicament according to Claim 8, **characterized in that** the neurodegenerative disease is either Alzheimer's disease, Parkinson's disease, frontoparietal dementia, corticobasal degeneration or Pick's disease.

10. Process for preparing compounds of formula (I) as defined in Claim 1 and for which R3 is (1-6C)alkyl, aryl(1-6C)alkyl, heteroaryl(1-6C)alkyl, heterocycloalkyl, cycloalkyl or polycycloalkyl, these radicals being optionally substituted with one or more substituents chosen from halogen, CN, NO₂, NH₂, OH, OR1, COOH, C(O)OR1, -O-C(O)R1, NR1R2, -NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, aryl, heteroaryl, formyl, trifluoromethyl, trifluoromethylsulfanyl, trifluoromethoxy and (1-6C)alkyl, and R1 and R2 are, independently of each other, a hydrogen, (1-6C)alkyl, aryl, alkenyl, alkynyl or heteroaryl, which are themselves optionally substituted with one or more substituents chosen from halogen, (1-6C)alkyl, (1-6C)alkoxy, CN, NO₂, NH₂, OH, COOH, COOalkyl, CONH₂, formyl, trifluoromethyl and trifluoromethoxy; starting with a derivative of formula (I) in which R3 is H, a derivative R1CHO and sodium triacetoxyborohydride in dichloromethane, and optionally conversion of the product obtained into a pharmaceutically acceptable salt.

11. Process for preparing the compounds of formula (I) as defined in Claim 1 and for which R3 is CONR1R2, R1 and R2 are, independently of each other, a hydrogen, (1-6C)alkyl, aryl, alkenyl, alkynyl or heteroaryl, which are themselves optionally substituted with one or more substituents chosen from halogen, (1-6C)alkyl, (1-6C)alkoxy, CN, NO₂, NH₂, OH, COOH, COOalkyl, CONH₂, formyl, trifluoromethyl and trifluoromethoxy, starting with OCNR1 and a derivative of formula (I) in which R3 is H, in tetrahydrofuran, optionally conversion of the product obtained into a pharmaceutically acceptable salt.

12. Process for preparing the compounds of formula (I) as defined in Claim 1 and for which R3 is SO₂R1 and R1 is a hydrogen, (1-6C)alkyl, aryl, alkenyl, alkynyl or heteroaryl, which themselves optionally substituted with one or more substituents chosen from halogen, (1-6C) alkyl, (1-6C)alkoxy, CN, NO₂, NH₂, OH, COOH, COOalkyl, CONH₂, formyl, trifluoromethyl and trifluoromethoxy, starting with a sulfonyl chloride R1SO₂Cl and a derivative of formula (I) in which R3 is H, in dichloromethane in the presence of a base, and optionally conversion of the compound obtained into a pharmaceutically acceptable salt.

13. As an intermediate product:
3-amino-5-phenyl-6-chloro-1-(2-trimethylsilylethoxy)-methyl]indazole.

## Patentansprüche

1. Verbindungen der Formel (I), worin: 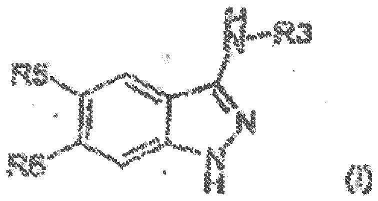
R3 für einen (C1-6)-Alkylrest, einen Arylrest, einen Aryl- (C1-6) -alkylrest, einen Heteroarylrest, einen Heteroaryl- (C1-6) -alkylrest, einen Aryl,-oder Heteroarylrest,, der mit einem (C1-10)-Cycloalkyl kondensiert ist, einen Heterocyclylrest, einen Heterocycloalkylrest, einen Cycloalkylrest, einen Adamantylrest, einen Polycycloalkylrest, einen Alkenylrest, einen Alkinylrest, einen CONR1R2-Rest, einen CSNR1R2-Rest, einen COOR1-Rest, einen SO₂R1-Rest oder einen C(=NH)NR1-Rest steht; wobei diese Reste gegebenenfalls durch 1 oder mehrere Substituenten, die unter Halogen, CN, NO₂, OH, OR1, COOH, C(O)OR1, -O-C(O)R1, NR1R2, NHC(O)R1, C(O)NR1R2, SR1, S(O)R1, SO₂R1, NHSO₂R1; SO₂NR1R2, C(S)NR1R2, NHC(S)R1, -O-SO₂R1, -SO₂-O-R1, Aryl, Heteroaryl, Formyl, Oxo, Trifluormethyl, Trifluormethylsulfanyl, Trifluormethoxy und (C1-6)-Alkyl ausgewählt sind, substituiert sind;
5 für ein Aryl steht, das gegebenenfalls durch 1 oder mehrere Substituenten, die unter Halogen, CN, NO₂, NH₂, OH, OR10, COOH, C(O)OR10, -O-C(O)R10, NR10R11, NHC(O)R10, C(O)NR10R11, NHC(S)R10, C(S)N10R11, SR10, S(O)R10, SO₂R10, NHSO₂R10, SO₂NR10R11, -O-SO₂R10, -SO₂-O-R10, Aryl, Heteroaryl, Formyl, Trifluormethyl, Trifluormethoxy und (C1-6)-Alkyl ausgewählt sind, substituiert ist;
R6 für einen Halogen-, Methyl-, Cyclopropyl-, CN-, OH-, Methoxy-, Trifluormethyl-, Methylenyl-, Acetylenyl-, Trifluormethoxy-, NO₂ NH₂- oder NMe₂-Rest steht;
R1, R2, R10 und R11 unabhängig voneinander für Wasserstoff, (C1-6)-Alkyl, Aryl, Alkenyl, Alkinol oder Heteroaryl stehen, wobei diese Reste selbst gegebenenfalls durch 1 oder mehrere Substituenten, die unter Halogen, (C1-6)-Alkyl, (C1-6)-Alkoxy, CN, NO₂, NH₂, OH, COOH, COO-Alkyl, CONH₂, Formyl, Oxo, Trifluormethyl und Trifluormethoxy ausgewählt sind, substituiert sind;
Racemate, Enantiomere und Diastereomere davon und Gemische davon, Tautomere davon und pharmazeutisch unbedenkliche Salze davon.

2. Verbindungen der Formel (I) nach Anspruch 1, worin: 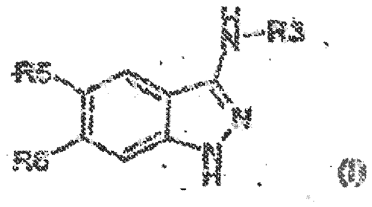
R3 für einen (C1-6)-Alkylrest, einen Arylrest, einen Aryl-(C1-6)-alkylrest, einen Heteroarylrest, einen Heteroaryl- (C1-6) -alkylrest, einen Aryl-oder Heteroarylrest, der mit einem (C1-10)-Cycloalkyl kondensiert ist, einen Heterocyclylrest, einen Heterocycloalkylrest, einen Cycloalkylrest, einen Adamantylrest, einen Polycycloalkylrest, einen Alkenylrest, einen Alkinylrest, einen CONR1R2-Rest, einen CSNR1R2-Rest, einen COOR1-Rest, einen SO₂R1-Rest oder einen C(=NH)NR1-Rest steht; wobei diese Reste gegebenenfalls durch 1 oder mehrere Substituenten, die unter Halogen, CN, NO₂, NH₂, OH, OR1, COOH, C(O)OR1, -O-C(O)R1, NR1R2, NHC(O)R1, C(O)NR1R2, SR1, S(O)R1, SO₂R1, NHSO₂R1, SO₂NR1R2, C(S) R1R2, NHC(S)R1, -O-SO₂R1, -SO₂-O-R1, Aryl, Heteroaryl, Formyl, Oxo, Trifluormethyl, Trifluormethylsulfanyl, Trifluormethoxy und (C1-6)-Alkyl ausgewählt sind, substituiert sind;
R5 für ein Phenyl steht, das gegebenenfalls durch 1 oder mehrere Substituenten, die unter Halogen, CN, NO₂, OH, OR10, COOH, C(O)OR10, -O-C(O)R10, NR10R11, NHC(O)R10, C(O)NR10R11, NHC (S) R10, C(S)NR10R11, SR10, S(O)R10, SO₂R10, NHSO₂R10, SO₂NR10R11, -O-SO₂R10, -SO₂-O-R10, Aryl, Heteroaryl, Formyl, Trifluormethyl, Trifluormethoxy und (C1-6)-Alkyl ausgewählt sind, substituiert ist;
R6 für einen Halogen-, Methyl-, Cyclopropyl-, CN-, OH-, Methoxy-, Trifluormethyl-, Methylenyl-, Acetylenyl-, Trifluormethoxy-, NO₂ NH₂- oder NMe₂-Rest steht;
R1, R10 und R11 unabhängig voneinander für Wasserstoff, (C1-6)-Alkyl, Aryl, Alkenyl, Alkinol oder Heteroaryl stehen, wobei diese Reste selbst gegebenenfalls durch 1 oder mehrere Substituenten, die unter Halogen, (C1-6)-Alkyl, (C1-6) -Alkoxy, CN, NO₂, NH₂, OH, COOH, COO-Alkyl, CONH₂, Formyl, Oxo, Trifluormethyl und Trifluormethoxy ausgewählt sind, substituiert sind;
Racemate, Enantiomere und Diastereomers davon und Gemische davon, Tautomere davon und pharmazeurisch unbedenkliche Salze davon.

3. Verbindung nach Anspruch 1, **dadurch gekennzeichnet, dass** sie unter
N-(Bicyclo[2.2.1]hept-5-en-2-ylmethyl)-6-chlor-5-phenyl-1H-indazol-3-amin,
6-Chlor-N-(3,3-dimethylbutyl)-5-phenyl-1H-indazol-3-amin,
6-Chlor-N-(3-phenylpropyl)-5-phenyl-1H-indazol-3-amin,
6-Chlor-N-(cyclopropylmethyl)-5-phenyl-1H-indazol-3-imin,
6-Chlor-N-(cyclopentylmethyl)-5-phenyl-1H-indazol-3-amin,
6-Chlor-N-[3-(methylthio)propyl]-5-phenyl-1H-indazol-3-Amin,
6-Chlor-N-(phenylethyl)-5-phenyl-1H-indazol-3-amin,
6-Chlor-N-(cyclohexylmethyl)-5-phenyl-1H-indazol-3-amin,
6-Chlor-N-propyl-5-phenyl-1H-indazol-3-amin,
6-Chlor-N-(2,2,3,3,4,4,4-heptafluorbutyl)-5-phenyl-1H-indazol-3-amin-hydrat,
6-Chlor-N-(4,4,4-trifluorbutyl) -5-phenyl-1H-indazol-3-amin,
6-Chlor-N-[(4-methoxyphenyl)methyl]-5-phenyl-1H-indazol-3-amin,
6-Chlor-N-(phenylmethyl)-5-phenyl-1H-indazol-3-amin,
6-Chlor-N-[(4-cyanophenyl)methyl]-5-phenyl-1H-indazol-3-amin,
N-[(4-Chlorphenyl)methyl] -6-chlor-5-phenyl-1H-indazol-3-amin,
6-Chlor-N-[(3-methoxyphenyl)methyl]-5-phenyl-1H-indazol-3-amin,
6-Chlor-N-{[4-(trifluormethoxy)phenyl]methyl]-9-phenyl-1H-indazol-3-amin,
N-[4-[[[6-Chlor-5-phenyl-1H-indazol-3-yl]amino]methyl]phenyl]acetamid,
6-Chlor-N-[(3,5-dichlorphenyl)-methyl]-5-phenyl-1H-indazol-3-amin,
6-Chlor-5-phenyl-N-[[4-(trifluormethyl)phenyl]-methyl]-1H-indazol-3-amin,
6-Chlor-N-[(4-fluorphenyl)methyl]-5-phenyl-1H-indazol-3-amin,
6-Chlor-N-[3-(4-methylphenoxy)phenylmethyl]-5-phenyl-1H-indazol-3-amin,
N-(2,2,3,3,4,4,4-Heptafluorbutyl)-6-chlor-5-phenyl-1H-indazol-3-amin,
6-Chlor-5-phenyl-N-[[3,5-bis(trifluormethyl)-phenyl]methyl]-1H-indazol-3-amin,
6-Chlor-5-phenyl-N-[[3-(trifluormethyl)phenyl]-methyl]-1H-indazol-3-amin,
6-Chlor-N-[(6-methoxy-2-naphthalinyl)methyl]-5-phenyl-1H-indazol-3-amin,
6-Chlor-N-[(pentafluorphenyl)methyl]-5-phenyl-1H-indazol-3-amin,
6-Chlor-N-[[4-(methylthio)phenyl]methyl]-5-phenyl-1H-indazol-3-amin,
N-[(4-Chlor-3-fluorphenyl)methyl]-6-chlor-5-phenyl-1H-indazol-3-amin,
6-Chlor-5-phenyl-N-(3,3,3-trifluorpropyl)-1H-indazol-3-amin,
6-Chlor-5-phenyl-N-(3-thienylmethyl)-1H-indazol-3-amin,
N-(Bicyclo[2.2.1]hept-5-en-2-ylmethyl)-6-chlor-5-phenyl-1H-indazol-3-amin,
N-(1,1'-Biphenyl-4-ylmethyl)-6-chlor-5-phenyl-1H-indazol-3-amin,
6-Chlor-N-[[4-(dimethylamino)phenyl]methyl]-5-phenyl-1H-indazol-3-amin,
N-(2,2'-Bithiophen-5-ylmethyl)-6-chlor-5-phenyl-1H-indazol-3-amin,
6-Chlor-5-phenyl-N-[[1-(phenylmethyl)-1H-imidazol-2-yl]methyl]-1H-indazol-3-amin,
6-Chlor-N-[[1-methyl-1H-imidazol-2-yl]methyl]-5-phenyl-1H-indazol-3-amin,
6-Chlor-N-[(1-methyl-1H-indol-3-yl)methyl]-5-phenyl-1H-indazol-3-amin,
6-Chlor-N-[(5-methyl-2-furanyl)methyl]-5-phenyl-1H-indazol-3-amin,
6-Chlor-5-phenyl-N-(1H-pyrrol-2-ylmethyl)-1H-indazol-3-amin,
6-Chlor-5-phenyl-N-[(1H-imidazol-2-yl)methyl]-1H-indazol-3-amin,
6-Chlor-5-phenyl-N-[(1H-imidazol-4-yl)methyl]-1H-indazol-3-amin,
6-Chlor-5-phenyl-N-(1H-pyrazol-3-ylmethyl)-1H-indazol-3-amin,
6-Chlor-N-[[2-methyl-1H-imidazol-4-yl]methyl]-5-phenyl-1H-indazol-3-amin,
6-Chlor-N-[(3,5-dimethyl-1-phenyl-1H-pyrazol-4-yl)methyl]-5-phenyl-1H-indazol-3-amin,
6-Chlor-5-phenyl-N-[[2-phenyl-1H-imidazol-4-yl]-methyl]-1H-indazol-3-amin,
6-Chlor-N-[[5-4-chlorphenyl)-2-furanyl]methyl]-5-phenyl-1H-indazol-3-amin,
6-Chlor-5-phenyl-N-[(1-methyl-1H-pyrrol-2-yl)-inethyl]-1H-indazol-3-amin,
4-[5-[[[6-Chlor-5-phenyl-indazol-3-yl]amino]-methyl]-2-furanyl]-benzolsulfonamid,
6-Chlor-5-phenyl-N-(3-thienylmethyl)-1H-indazol-3-amin,
6-Chlor-5-phenyl-N-[[2-phenyl-1H-imidazol-4-yl]-methyl]-1H-indazol-3-amin,
2-[[[6-Chlor-5-phenyl-1H-indazol-3-yl]amino]methyl]-5-(methylthio)-1H-imidazo1-4-carbonsäureeethylester,
6-Chlor-5-phenyl-N-[[5-[4-(trifluormethyl) phenyl]-2-furanyl]methyl]-1H-indazol-3-amin,
6-Chlor-5-phenyl-N-[2-(1-piperidinyl)ethyl]-1H-indazol-3-amin,
6-Chlor-N-[2-(4-morpholinyl)ethyl]-5-phenyl-1H-indazol-3-amin,
N-(6-chlor-5-phenyl-1H-indazol-3-yl)-N'-(3,5-dichlorphenyl)harnstoff,
N-(6-Chlor-5-phenyl-1H-indazol-3-yl)-N'-(2-propenyl)harnstoff,
N-(6-Chlor-5-phenyl-1H-indazol-3-yl)-N'-(phenylmethyl)harnstoff,
N-(-Chlor-5-enyl-1-in azol-3-yl)-N'-(4-phenoxyphenyl)harnstoff,
N-(6-Chlor-5-phenyl-1H-indazol-3-yl)-N'-(4-methoxyphenyl)methyl]harnstoff,
N-(6-Chlor-5-phenyl-1H-indazol-3-yl)-N'-[4-(trifluormethyl)phenyl]harnstoff,
N-(6-Chlor-5-phenyl-1H-indazol-3-yl)-N'-(4-methoxyphenyl)harnstoff,
N-(6-Chlor-5-phenyl-1H-indazol-3-yl)-N'-cyclo-hexylharnstoff,
N-(6-Chlor-5-phenyl-1H-indazol-3-yl)-N'-propyl-harnstoff,
N-(6-Chlor-5-phenyl-1H-indazol-3-yl)-N'-(4-chlorphenyl)harnstoff,
N-(6-Chlor-5-phenyl-1H-indazol-'3-yl)-N'-(4-fluorphenyl)harnstoff,
N-[6-Chlor-5-phenyl-1H-indazol-3-yl]-N'-(tricyclo[3.3.1.1^{3,7} ]dec)-1-ylharnstoff,
N-(6-Chlor-5-phenyl-1H-inazol-3-yl)-N'-(4-methylphenyl)harnstoff,
N-[6-Chlor-5-phenyl-1H-indazol-3-yl]-4-methyl-benzolsulfonamid,
N-[6-Chlor-5-phenyl-1H-indazol-3-yl]methansulfon-amid,
N-[6-Chlor-5-phenyl-1H-indazol-3-yl]-2-propan-sulfonamid,
N-[6-Chlor-5-phenyl-1H-indazol-3-yl]-2,2,2-tri-fluorethansulfonamid,
N-[6-Chlor-5-phenyl-1H-indazol-3 -yl]-2-thiophen-sulfonamid,
N-[6-Chlor-,5-phenyl-1H-indazol-3-yl]benzolsulfonamid,
N-[6-Chlor-5-phenyl-1H-indazol-3-yl] 4-(trifluormethyl)benzolsulfonamid,
N-[6-Chlor-5-phenyl-1H-indazöl-3-yl]-5-(3-isoxazolyl)-2-thiophensulfonamid,
N-[6-Chlor-B-phenyl-1H-indazol-3-yl]-4-fluor-benzolsulfonamid,
N-[6-Chlor-5-phenyl-1H-indazol-3-yl]4-methoxy-benzolsulfonamid,
N-[6-Chlor-5-phenyl-1H-indazol-3-yl]benzolmethan-sulfonamid,
N-[6-Chlor-5-phenyl-1H-indazol-3-yl]-1-methyl-1H-imidazol-4-sulfonamid,
N-[6-Chlor-5-phenyl-1H-indazol-3-yl]-4-(1,1-dimethylethyl)benzolsulfonamid,
N-[4-[[(6-Chlor-5-phenyl-1H-indazol-3-yl)amino]-sulfonyl]phenyl]acetamid,
N-[6-Chlor-5-phenyl-1H-indazol-3-yl]-4-methyl-benzolmethansulfonamid,
6-chlor-N-(pentafluorphenyl)-5-phenyl-1H-indazol-3-amin,
6-Chlor-N-(3,4-difluorphenyl)-5-phenyl-1H-indazol-3-amin,
6-Chlor-5-phenyl-N-(2,3,5,6-tetrafluorphenyl)-1H-indazol-3-amin,
6-Chlor-5-phenyl-N-(2,4,6-trifluorphenyl)-1H-indazol-3-amin,
6-Chlor-N-(4-fluorphenyl)-5-phenyl-1H-indazol-3-amin,
6-Chlor-N-[3-{trifluormethyl)phenyl]-5-phenyl-1H-indazol-3-amin,
6-Chlor-N-[4-(trifluormethyl)phenyl]-5-phenyl-1H-indazol-3-amin,
6-Chlor-N- [3-fluor-5-(trifluormethyl)phenyl]-5-phenyl-1H-indazol-3-amin,
6-Chlor-N-(4-nitrophenyl)-5-phenyl-1H-indazol-3-amin,
6-Chlor-N-(3-nitrophenyl)-5-phenyl-1H-indazol-3-amin,
6-Chlor-N-(3-methoxyphenyl)-5-phenyl-1H-indazol-3-amin,
6-Chlor-N-(4-methoxyphenyl)-5-phenyl-1H-indazol-3-amin,
6-Chlor-N,5-diphenyl-1H-indazol-3-amin,
6-Chlor-N-(1-pyridinyl)-5-phenyl-1H-indazol-3-amin,
6-Chlor-N-(2-pyridinyl)-5-phenyl-1H-indazol-3-amin,
Racematen, Enantiomeren und Diastereomeren davon und Gemischen davon, Tautomeren davon und pharmazeutisch unbedenklichen Salzen davon aus gewählt ist.

4. Verbindung nach einem der Ansprüche 1 bis 2, **dadurch gekennzeichnet**, sie unter
N-Butyl-6-chlor-5-phenyl-1H-indazol-3-amin,
3-(6-Chlor-5-phenyl-1H-indazol-3-ylamino)thiophen-2-carbonitril,
(6-Chlor-5-phenyl-1H-indazol-3-yl)(pyridin-2-yl)-amin,
(6-Chlor-5-phenyl-1H-indazol-3-yl)(5-nitropyridin-2-yl)amin,
(6-Chlor-5-phenyl-1H-indazol-3-yl)(6-methoxy-pyridin-2-yl)amin,
N-(6-Chlor-5-phenyl-1H-indazol-3-yl)-N'-phenylharnstoff,
1-(6-Chlor-5-phenyl-1H-indazol-3-yl)-3-(4-ethoxyphenyl)harnstoff,
1-(6-Chlor-5-phenyl-1H-indazol-3-yl)-3-(3,4-dichlorphenyl)harnstoff,
3-[3-(6-Chlor-5-phenyl-1H-indazol-3-yl)ureido]-propionsäuremethylester,
1-(6-Chlor-5-phenyl-1H-indazol-3-yl)-3-(4-(dimethylamino)phenyl)harnstoff,
1-(6-Chlor-5-phenyl-1H-indazol-3-yl)-3-isopropyl-harnstoff,
1-(6-Chlor-5-phenyl-1H-indazol-3-yl)-3-cyclohexyl-harnstoff,
1-(6-Chlor-5-phenyl-1H-indazol-3-yl)-3-(3-(trifluormethyl)phenyl)harnstoff,
1-(6-Chlor-5-phenyl-1H-indazol-3-yl)-3-(2-(thiophen-2-yl)ethyl)harnstoff,
1-(1,3-Benzodioxol-5-yl)-3-(6-chlor-5-phenyl-1H-indazol-3-yl)harnstoff,
1-(6-Chlor-5-phenyl-1H-indazol-3-yl)-3-(3,5-dimethylisoxazol-4-yl)harnstoff,
1-Benzyl-3-(6-chlor-5-phenyl-1H-indazol-3-yl)harnstoff,
1-(6-Chlor-5-phenyl-1H-indazol-3-yl)-3-(phenethyl)thioharnstoff,
1-(6-Chlor-5-phenyl-1H-indazol-3-yl)-3-[3-(4-methylpiperazin-1-yl)propyl]harnstoff,
1-(6-Chlor-5-phenyl-1-indazol-3-yl)-3-(3-(imidazol-1-yl)propyl)harnstoff,
1-(6-Chlor-5-phenyl-1H-indazol-3-yl)-3-(2-hydroxyethyl)harnstoff,
1-(6-Chlor-5-phenyl-1H-indazol-3-yl)-3-[3-(4-methylpiperazin-1-yl)propyl]harnstoff,
(6-Chlor-5-phenyl-1H-indazol-3-yl)carbamidsäuremethylester,
(6-Chlor-5-phenyl-1H-indazol-3-yl)harnstoff,
(6-Chlor-5-phenyl-1H-indazol-3-yl) carbamidsäurebenzylester,
(6-Chlor-5-phenyl-1H-indazol-3-yl) carbamidsäureallylester,
(6-Chlor-5-phenyl-1H-indazol-3-yl) carbamidsäureisobutylester,
1-(3-(Azetidin-1-yl)propyl)-3-(6-chlor-5-phenyl-1H-indazol-3-yl)harnstoff,
1-(6-Chlor-5-phenyl-1H-indazol-3'-yl)-3-(3-chlorpropyl)harnstoff,
1-(6,7-Difluor-5-phenyl-1H-indazol-3-yl)-3-(3-(imidazol-1-yl)propyl)harnstöff,
1-(3-Aminopropyl)-3-(6-chlor-5-phenyl-1H-indazol-3-yl)harnstoff,
1-(6-Chlor-5-phenyl-1H-indazol-3-yl)-3-[4-(4-(pyridin-3-yl)imidazol-1-yl)butyl]harnstoff,
1-(6-Chlor-5-phenyl-1H-indazol-3-yl)-3- (2-(pyrrolidin-1-yl)ethyl)harnstoff,
N-(6-Chlor-5-phenyl-1H-indazol-3-yl)-3-methoxybenzolsulfonamid,
Racematen davon, Enantiomeren davon, Tautomeren davon sowie pharmazeutisch unbedenklichen Salzen davon ausgewählt ist.

5. Verbindung nach einem Ansprüche 1 bis 4, die zur Herstellung eines Arzneimittels verwendet wird.

6. Pharmazeutische Zusammensetzung, **dadurch gekennzeichnet, dass** sie in einem pharmazeutisch unbedenklichen Medium eine Verbindung gemäß einem der Ansprüche 1 bis 4 enthält.

7. Arzneimittel, das in eine pharmazeutisch unbedenklichen Medium mindestens eine Verbindung gemäß einem der Ansprüche 1 bis 4 enthält, zur therapeutischen Anwendung bei der Behandlung von Erkrankungen, bei denen eine Phosphorylierung des Tau-Proteins beobachtet wird.

8. Arzneimittel nach Anspruch 7, **dadurch gekennzeichnet, dass** es mindestens eine Verbindung gemäß einem der Ansprüche 1 bis 4 enthält, zur therapeutischen Anwendung bei der Behandlung von neurodegenerativen Erkrankungen, Schlaganfällen, Schädel- und Rückenmarkstraumata und peripheren Neuropathien, Obesitas, Stoffwechselerkrankungen, Typ-II-Diabetes, essentieller Hypertonie, atherosklerotischen Herz-Kreislauf-Erkrankungen, Syndrom der polyzystischer Ovarien, Syndrom X, Immundefekt und Krebs.

9. Arzneimittel nach Anspruch 8, **dadurch gekennzeichnet**, ass es sich bei der neurodegenerativen Erkrankung um Alzheimer-Krankheit, Parkinson-Krankheit, frontoparietale Demenz, kortikobasale Degeneration oder Pick-Krankheit handelt.

10. Verfahren zur Herstellung der Verbindungen der Formel (I) Anspruch 1, für die R3 für (C1-6)-Alkyl, Aryl-(C1-6)-alkyl, Heteroaryl-(C1-6)-alkyl, Heterocycloalkyl, Cycloalkyl oder Polycycloalkyl steht, wobei diese Reste gegebenenfalls durch 1 oder mehrere unter Halogen, CN, NO₂, NH₂, OH, OR₁, COOH, C(O)OR1, -O-C(O)R1, NR1R2, NHC(O)R1, C(O)NR1R2, SR1, S(O)R1, SO₂R1, NHSO₂R1, SO₂NR1R2, C(S)NR1R2, NHC(S)R1, -O-SO₂R1, -SO₂-O-R1, Aryl, Heteroaryl, Formyl,- Trifluormethyl, Trifluormethylsulfanyl, Trifluormethoxy und (Cl-6)-Alkyl ausgewählte Substituenten substituiert sind; und R1 und R2 unabhängig voneinander für Wasserstoff, (C1-6) -Alkyl, Aryl, Alkenyl, Alkinol oder Heteroaryl stehen, wobei diese Reste selbst gegebenenfalls durch 1 oder mehrere unter Halogen, (C1-6) -Alkyl, (C1-6)-Alkoxy, CN, NO₂, NH₂, OH, COOH, COO-Alkyl, CONH₂, Formyl, Trifluormethyl und Trifluormethoxy ausgewählte Substituenten substituiert sind; aus einem Derivat der Formel (I), worin R3 für H steht, einem RICHO-Derivat und Natriumtriacetoxyborhydrid in Dichlormethan, und gegebenenfalls Umwandlung des erhaltenen Produkts in ein pharmazeutisch unbedenkliches Salz.

11. Verfahren zur Herstellung der Verbindungen der Formel (I) gemäß Anspruch 1, für die R3 für CONR1R2 steht und R1 und R2 unabhängig voneinander für Wasserstoff, (C1-6)-Alkyl, Aryl, Alkenyl, Alkinyl oder Heteroaryl stehen, wobei diese Reste selbst gegebenenfalls durch 1 oder mehrere unter Halogen, (C1-6)-Alkyl, (C1-6)- Alkoxy, CN, NO₂, NH₂, OH, COOH, COO-Alkyl, CONH₂, Formyl, Trifluormethyl und Trifluormethoxy ausgewählte'Substituenten substituiert sind, stehen; aus OCNR1 und einem Derivat der Formel (I), worin R3 für H steht, in Tetrahydrofuran, und gegebenenfalls Umwandlung des erhaltenen Produkts in ein pharmazeutisch unbedenkliches Salz.

12. Verfahren zur Herstellung der Verbindungen der Formel (I) Anspruch 1, für die R3 für SO₂R1 steht und R1 für Wasserstoff, (C1-6)-Alkyl, Aryl, Alkenyl, Alkinyl oder Heteroaryl steht, wobei diese Reste selbst gegebenenfalls durch 1 oder mehrere unter Halogen, (C1-6)-Alkyl, (C1-6)-Alkoxy, CN, NO₂, NH₂, OH, COOH, COO-Alkyl, CONH₂, Formyl, Trifluormethyl und Trifluormethoxy ausgewählte Substituenten substituiert sind; aus einem Sulfonylchlorid R1SO₂Cl und einem Derivat der Formel (I), worin R3 für H steht, in Dichlormethan in Gegenwart einer Base, und gegebenenfalls Umwandlung der erhaltenen Verbindung in ein pharmazeutisch unbedenkliches Salz.

13. Als Zwischenprodukt
3-Amino-5-phenyl-6-chlor-1-(2-trimethylsilyl-ethoxy)methyl]indazol.
